# EUROPEAN PATENT SPECIFICATION

(11) **EP 1 147 416 B1**
(45) Date of publication and mention of the grant of the patent: **29.05.2013**
(21) Application number: 00911609.6
(22) Date of filing: 21.01.2000
(51) Int. Cl.: B82Y 5/00, B82Y 15/00, B82Y 30/00, G01N 33/543

(54) **ASSAYS INVOLVING COLLOIDS AND NON-COLLOIDAL STRUCTURES**
ASSAYS UNTER VERWENDUNG VON KOLLOIDEN SOWIE NICHT-KOLLOIDALEN STRUKTUREN
INTERACTION ENTRE DES ESPECES IMMOBILISEES SUR DES COLLOIDES ET DES ESPECES EXISTANT SUR DES STRUCTURES COLLOIDALES

(30) Priority: 23.01.1999 US 116975 P; 03.05.1999 US 132289 P; 07.05.1999 US 133148 P; 12.05.1999 US 133772 P
(43) Date of publication of application: 24.10.2001
(60) Divisional application: 10184879.4
(73) Proprietor: Minerva Biotechnologies Corporation, Newton, MA 02458 (US)
(72) Inventor: BAMDAD, Cynthia, Carol, Newton, MA 02458 (US); BAMDAD, R., Shoshana, Newton, MA 02458 (US)
(74) Representative: Killin, Stephen James
(86) International application number: PCT/US2000/001504
(87) International publication number: WO 2000/043783

(56) References cited:
- EP-A- 0 579 343
- EP-A1- 0 317 001
- WO-A1-94/09117
- WO-A1-95/24641
- WO-A1-97/34150
- WO-A1-98/04740
- WO-A1-99/01766
- WO-A2-98/31839
- WO-A2-98/35232
- DE-A- 3 806 558
- GB-A- 2 095 258
- US-A- 4 313 734
- US-A- 4 945 045
- US-A- 4 978 610
- US-A- 5 079 172
- US-A- 5 620 850
- G A ROBINSON, H A O HILL, R D PHILO, J M GEAR, S J RATTLE, G C FORREST: "Bioelectrochemical Enzyme Immunoassay of Human Choriogonadotropin with Magnetic Electrodes" CLINICAL CHEMISTRY, vol. 31, no. 9, 1985, pages 1449-1452, XP002142426
- CHOI MYUNG JA; KIM SO YOUNG; CHOI JEONGEUN; PAENG INSOOK RHEE: "Labeling digoxin antibody with colloidal gold and ferrocene for its use in a membrane immunostrip and immunosensor" MICROCHEMICAL JOURNAL, vol. 63, September 1999 (1999-09), pages 92-99, XP000924842
- FAN ET AL: "Adsorption of Surface-Modified Colloidal Gold Particles onto Self - Assembled Monolayers: A Model System for the Study of Interactions of Colloidal Particles and Organic Surfaces", LANGMUIR: THE ACS JOURNAL OF SURFACES AND COLLOIDS, AMERICAN CHEMICAL SOCIETY, WASHINGTON, DC, USA, vol. 13, no. 2, 1 January 1997 (1997-01-01), pages 119-121, XP002159017, ISSN: 0743-7463, DOI: 10.1021/LA960814M
- INGRAM R S ET AL: "POLY-HETERO-OMEGA-FUNCTIONALIZED ALKANETHIOLATE-STABILIZED GOLD CLUSTER COMPOUNDS", JOURNAL OF THE AMERICAN CHEMICAL SOCIETY, AMERICAN CHEMICAL SOCIETY, WASHINGTON, DC; US, vol. 119, no. 39, 1 May 1997 (1997-05-01), pages 9175-9178, XP002936276, ISSN: 0002-7863, DOI: 10.1021/JA971734N
- ELGHANIAN R ET AL: "SELECTIVE COLORIMETRIC DETECTION OF POLYNUCLEOTIDES BASED ON THE DISTANCE-DEPENDENT OPTICAL PROPERTIES OF GOLD NANOPARTICLES", SCIENCE, AMERICAN ASSOCIATION FOR THE ADVANCEMENT OF SCIENCE, WASHINGTON, DC; US, vol. 277, 22 August 1997 (1997-08-22), pages 1078-1081, XP002916633, ISSN: 0036-8075, DOI: 10.1126/SCIENCE.277.5329.1078
- TEMPLETON A C ET AL: "Gateway Reactions to Diverse, Polyfunctional Monolayer-Protected Gold Clusters", JOURNAL OF THE AMERICAN CHEMICAL SOCIETY, AMERICAN CHEMICAL SOCIETY, WASHINGTON, DC; US, vol. 120, 1 January 1998 (1998-01-01), pages 4845-4849, XP002276844, ISSN: 0002-7863, DOI: 10.1021/JA980177H
- AKIRA YOSHIZUMI ET AL: 'Self-assembled monolayer of sugar-carrying polymer chain: Sugar balls from 2-methacryloyloxyethyl D-glucopyranoside' LANGMUIR vol. 15, no. 2, 01 January 1999, pages 482 - 488, XP055014678 DOI: 10.1021/la980374u ISSN: 0743-7463
- DAMRONGCHAI N ET AL: "Self-assembling of glutathione S-transferase/calmodulin fusion protein on chemically modified gold surface.", JOURNAL OF BIOTECHNOLOGY 13 JUN 1997 LNKD- PUBMED:9232034, vol. 55, no. 2, 13 June 1997 (1997-06-13), pages 125-133, ISSN: 0168-1656
- DANIEL M-C ET AL: "GOLD NANOPARTICLES: ASSEMBLY, SUPRAMOLECULAR CHEMISTRY, QUANTUM-SIZE-RELATED PROPERTIES, AND APPLICATIONS TOWARD BIOLOGY, CATALYSIS, AND NANOTECHNOLOGY", CHEMICAL REVIEWS, AMERICAN CHEMICAL SOCIETY, US, vol. 104, no. 1, 1 January 2004 (2004-01-01), pages 293-346, XP001186731, ISSN: 0009-2665, DOI: 10.1021/CR030698+
- CARL S. WEISBECKER ET AL: 'Molecular Self-Assembly of Aliphatic Thiols on Gold Colloids' LANGMUIR vol. 12, no. 16, 01 January 1996, pages 3763 - 3772, XP055041069 DOI: 10.1021/la950776r ISSN: 0743-7463
- LON A. PORTER ET AL: 'Gold and Silver Nanoparticles Functionalized by the Adsorption of Dialkyl Disulfides' LANGMUIR vol. 14, no. 26, 01 December 1998, pages 7378 - 7386, XP055030888 DOI: 10.1021/la980870i ISSN: 0743-7463

## Description

### Field of the invention

This application relates generally to chemical and biochemical and the detection methods, and more particularly to techniques in which colloids bind to non-colloidal structures such as electrodes, beads, or cells, and to techniques in which multiple signals indicate a single binding event. Techniques including drug screening are facilitated by the application.

### Background of the invention

Drug discovery is facilitated by screening large numbers of candidate compounds for interaction with target receptors under physiological conditions. Of particular importance are cell surface receptors. Many of the biomolecular interactions that promote tumorigenesis involve cell surface proteins that mediate both intra- and intercellular signaling. "Tumor markers" are molecules on the surface of a cell that are either exclusively expressed, or over-expressed, as a result of transformation to a neoplastic state. Some of these markers have been correlated with the ability of the tumor to invade tissues and display an aggressive course of growth characterized by metastases (these tumors generally are associated with a poor outcome for the patient). For example, the interaction between the cell surface receptor αVβ3 and the cell adhesion molecule vitronectin has been implicated in angiogenesis. *See* J. Varner et al., "Integrins and cancer," Curr Opin Cell Biol. 8:724 (1996); B. Vailhe et al., "In vitro angiogenesis is modulated by the mechanical properties of fibrin gels and is related to a αVβ3 integrin localization," In Vitro Cell Dev Biol Anim., 33:763 (1997); M. Horton, "The αVβ3 integrin'vitronectin receptor'," Int J Biochem Cell Biol, 29:721 (1997). Indeed, the increased concentration of this receptor on a melanoma cell has been correlated with a poor prognosis. Another example is the MUC-1 antigen; this antigen is overexpressed on breast, prostate, lung and ovarian cancers.

The ability to detect interactions between ligands and target receptors on the surface of live, intact cells, would enable the screening of candidate compounds to disrupt these interactions. Screening compound libraries for drugs that inhibit the action of cell surface receptors depends critically on the receptors being in their native conformation and multimerization state throughout the drug screening process. According to current technologies, it is difficult or impossible to detect the interaction of cell surface receptors with their natural ligands.

Current technologies are limited in their ability to assay ligand interactions on cells. One such technology is an ELISA assay. In an ELISA, cells are first adhered to a 96-well plastic plate, either via an antibody, collagen, or direct non-specific interaction with the plastic plate. A cognate antibody is then allowed to bind to the cell surface protein of interest. To detect the interacting complex, a second interspecies antibody, designed to bind to the first antibody, and that has been conjugated to an enzyme (usually horseradish peroxidase or alkaline phosphatase), is added. Washing steps remove unbound antibodies. The enzyme's substrate is then added which will result in a colorimetric change if the enzyme is present. In a variation of the technique, the secondary antibody is conjugated to a fluorescent tag, which is then detected in a fluorescence reader. There are several inherent problems which limit the usefulness of this technique: 1) there is a high rate of non-specific antibody adsorption to the plastic; 2) weak ligand-receptor interactions are disrupted by the many washing steps that are required by this technique; 3) the frequent washing steps as well as the use of peroxide as an enzyme substrate can be toxic to cells; and 4) a large number of cells are required for the assay. Specifically, this assay is not ideally suitable for screening drug candidates for the following reasons: 1) the antibody-receptor interaction can be high affinity interactions and not likely to be disrupted by a small molecule or protein drug candidates; 2) the antibody may bind the target receptor at a site that does not disrupt the receptor's normal function; and 3) the assay gives no information as to cell viability in the presence of the drug.

WO990176 discloses mercaptosilane- stabilized metal nanoparticles and their use. The mercaptosilanes form a SAM on the metal nanoparticles with a size of 5nm or lower. Said nanoparticles can be conjugated to immunoreactive bioreagents such as antibodies, avidin and protein-A. Said molecules are covalently attached to a silane residue. An advantage of these silane-stabilized gold particles is the easy ability to bind more than one molecule simultaneously to the mercaptosilane shell of each gold particle.

What is needed is an approach for monitoring or controlling binding events between chemical or biological species that increases versatility and can increase sensitivity in a wide variety of specific interactions. For example, an approach for screening drugs against specific interactions of cell receptors and their ligands (including but not limited to the interaction of the (αVβ3 receptor) would be advantageous. In the latter example, the assay should be flexible so as to permit screening against a wide variety of tumor types, and particularly invasive tumors. Importantly, the screening approach should permit the rapid screening of large numbers of candidate drugs.

### Summary of the invention

The present application provides a variety of novel methods, compositions and species and articles for monitoring (detecting) interactions between chemical or biological species including techniques useful for drug screening.

The application provides a method in which a colloid particle is given the ability to fasten to a non-colloidal structure, and then whether the colloid particle has fastened to the non-colloidal structure is determined. This covers a wide range of interactions involving a colloid particles or, preferably, many colloid particles with a variety of structures. Non-colloidal structures including beads, such as magnetic beads, can interact with colloid particles as an aid in determining interaction of species immobilized with respect to the bead with species immobilized with respect to the colloid particles. The colloid particles can "decorate"the beads, rendering them visibly identifiable as an indication of a particular species/species interaction, can be used to recruit colloid particles to a surface where identification of the particles can be made through electronic or other means, and the like. Noncolloidal structures such as electrodes can act with colloid particles or species immobilized on the colloid particles to indicate a particular assay result. Non-colloidal structures such as cells can interact with colloid structures to indicate binding of the cell to a species carried by the colloid particle. Advantages of these and other techniques involving colloid particles and a noncolloid structure will become even more apparent from the description that follows.

The application provides a technique for linking a chemical or biological agent to an article, such as a bead, colloid particle or the like, via a metal binding tag/metal chelate linkage. Articles linked in this way to chemical or biochemical species are included in the invention.

The application involves a technique for the detection of ligand/protein interaction involving providing a ligand in fixed proximal relationship with an electroactive entity and determining interaction of the ligand with the protein by monitoring an electroactive signal given by the electroactive entity or the signal is dependent upon proximity of electroactive entity to the protein. This technique can be facilitated by an article which defines a surface, and a ligand suspected of interacting with a protein immobilized at the surface along with an electroactive entity which also is immobilized at the surface. The application facilitates a method for determining protein/ligand interaction in the absence of surface plasmon resonance without labeling either the protein or the ligand.

The application also provides techniques and components that allow for a method of signaling a single binding of a first biological or chemical agent to a second biological or chemical agent with a plurality of signaling entities. An article provided by the application facilitates this technique and it defines a first biological or chemical agent capable of biological or chemical binding to a second agent, linked to a plurality of signaling entities. The plurality of signaling entities can be linked to the agent by way of a polymer of dendrimer that carries a plurality of signaling entities and is adapted for linkage to an agent. The technique also can be facilitated by immobilizing the agent at a colloid particle at which a plurality of signaling entities are also immobilized. Preferably, more than three signaling entities signal a single chemical or biological binding event simultaneously. More preferably, at least 10, more preferably at least 50, and more preferably at least 1000 or 10,000 signaling entities signal a single chemical or biological binding event simultaneously according to this aspect of the application.

The application is defined by a colloid particle that has both a signaling entity and a protein immobilized thereupon. Techniques for use of this component also are included in the application.

The application also provides techniques for controlling the electronic permeability across a self-assembled monolayer at a surface. In the technique, self-assembled monolayer is formed upon a surface and is defined by a mixture of at least a first molecular species and a second molecular species. The first molecular species has a structure that promotes the self-assembly at the surface in a tightly-packed manner that prevents fluid to which the surface is exposed from communication electrically with the surface. That is, the first species forms a relatively tightly packed self-assembled monolayer (SAM) that seals the surface against fluids for the SAM is made up of only the first species. The SAM is made up of the first species and a second species that has a different molecular structure where the molecular structure causes disruption of the tightly-packed self-assembled structure thereby defining defects in the tightly-packed structure that allow fluid to which the surface is exposed to communicate electrically with the surface. In this context, the fluid communicates electrically with the surface by contacting the surface or coming in close enough proximity to the surface that electronic communication, via tunneling or the like, can occur. As will be seen from the description that follows, this can be very useful in conjunction with other techniques of the invention.

As mentioned, one feature of the application is that it provides a series of components and techniques for drug screening. The approach provides 1) a modular system for the attachment of natural ligands to universal signaling elements; 2) enhanced sensitivity of detection through the attachment of a plurality of signaling elements to each ligand; 3) a simpler format (without the need for washing steps, enzymatic cleavage and toxic substrates); 4) a convenient electronic output; and 5) the capability for multiplexing.

The present application contemplates immobilizing live, intact growing cells on conducting solid supports. Such cells, having both surface molecules (e. g. receptors) and intracellular signaling proteins, are attached to solid supports that can either be surfaces or particle-like in nature. Ligands (or"binding partners") to these cell-derived molecules, which can include both known and unknown ligands as well as putative drug candidates, are either unattached to other solid supports or attached to surfaces or particle-like structures, are allowed to interact with other cell-derived molecules in a manner such that binding between the two binding partners occurs and can be detected. One of the binding partners or its attached support can additionally be derivatized with a substance that can be recognized and quantitated by a detection apparatus. This complex (through interaction) is then brought into the presence of the detection apparatus using characteristics of the associated complex that differentiate it from the unassociated binding partners.

The cells are grown on a metal support (e. g. gold) that has been treated (e. g. coated with molecules compatible with cell growth) in a manner that preserves the conducting nature of the metal support. A homogeneous conducting layer of molecules may be attached to a metal surface that allows the conduction of electrons at a rate that is higher than a metal uniformly coated with insulating species of molecules (such as saturated alkyl thiolates). Alternatively, a heterogeneous layer of molecules including a mixture of first a second species that allow fluid exposed to the surface to communicate electrically with the surface as described above. The heterogeneous conducting layer of molecules may be attached to a metal surface; the heterogenous layer can be viewed as a lawn of low or non-conducting species (e. g. one such species or a plurality of such species) interrupted by conducting or high-conducting molecules (or"defects"in the lawn).

Preferably, in a heterogeneous conducting layer or heterogeneous layer allowing defects promoting fluid/surface electrocommunication, two types of molecules are attached to a metal surface: 1) alkyl thiol spacer molecules (terminated with either ethylene glycol units or methyl groups to inhibit non specific adsorption) are used to form the lawn and 2) a conducting species or defect-forming species. A conducting species can be readily identified by the experiments described below. The conducting species may contain at least one ring structure (e. g. a benzene ring, a pyrimidine ring, etc.). Alternatively, the conducting species contains no ring structure, but contains a group selected from-0-, -OH-, NH₂- and SH.

Preferably, in a homogeneous conducting layer, only one type of molecule is attached to a metal surface, i. e. a conducting species. Alternatively, a plurality of conducting species are also contemplated. Further description of defect-forming molecules and other aspects of this arrangement are described below.

As noted above, ligands (or "binding partners") to one or more cell-derived molecules can be attached to particle-like structures and employed in the assay. The ligands may be attached to colloids, said colloids comprising electroactive molecules (such as ferrocene). In such an assay, the interaction of the ligand with the cell-derived molecule (e.g. cell surface receptor) brings the colloid -- and the electroactive molecule -- near the metal surface, permitting electrodetection of the ligand interaction.

As noted above, the present application contemplates both methods and compositions. The present application contemplates a composition, comprising a first molecule and one or more signaling elements (*i.e.* molecules or clusters of molecules which provide a signal that can be detected by any means. including but not limited to, detection by change in light transmission, change in solution color, change in charge, etc.) attached to a solid support, wherein said first molecule is a ligand and is capable of interacting with a cell-surface receptor. Alternatively, the present application contemplates a composition, comprising a first molecule, a second molecule and a third molecule attached to a solid support, wherein said first molecule comprises a ligand capable of interacting with a cell-surface receptor, wherein said second molecule forms a monolayer on said solid support, and wherein said third molecule is electroactive.

The present application contemplates a variety of signaling elements, including but not limited to fluorescent molecules and enzymes capable of acting on colorimetric substrates. Preferably, the present application contemplates electroactive molecules that is, molecules having an oxidation/reduction potential that can be determined electronically proximate a working electrode of an appropriate, conventional electrical arrangement, as signaling elements. A preferred electroactive molecule is a metallocene. Metallocenes that can operate as electroactive signaling elements in accordance with the invention are known. One example of a particularly preferred electroactive molecule is one containing a ferrocene group, such as ferrocenyl thiol (C₃₅H₂₄FeS); however, other organic complexes of transitions metals are also contemplated as signaling elements.

It is not intended that the present application be limited by the nature of the chemical or biochemical. A wide variety of agents and binding partner of those agents such as antibody/antigen, antibody/hapten, enzyme/substrate, enzyme/inhibitor, enzyme/cofactor, binding protein/substrate, carrier protein/substrate, lectin/carbohydrate, receptor/hormone, receptor/effector, complementary strands of nucleic acid, protein/nucleic acid repressor/inducer, ligand/cell surface receptor, virus/ligand, etc., can be used for binding interactions of the application. The agent may be a ligand, specifically a peptide. Preferably, the peptide is derivatized with a moiety that can bind to a metal chelate (such as a histidine tag). It is convenient that the solid support comprise a metal chelate and said peptide is attached to said solid support via binding of said moiety to said metal chelate.

Cell-derived molecules, including both cell-surface receptors and intracellular signaling proteins, exist on or are attached to solid supports that can either be surfaces or particle-like in nature. Binding partners of these cell-derived proteins, which can include both known and unknown ligands as well as putative drug candidates, are attached to surfaces and/or particle-like structures, and are allowed to interact with the cell-derived proteins in a manner such that binding between the two binding partners occurs. One of the binding partners or its attached support can additionally be derivatized with a detectable substance. Interacting complexes are identified using characteristics of the associated complex that differentiate it from the unassociated binding partners. The presence of, or a change in, a detectable moiety, that is either co-immobilize with one of the binding partners on a common solid support or directly attached to one of the binding partners, is detected. Molecules that disrupt a relevant interaction can be identified by detecting a loss of this signal. Interacting partners are brought to a sensing apparatus by confining one of the binding partners to the sensing area and allowing it to recruit the other binding partner, or by manipulating characteristics of the associated complex that differentiate it from the unassociated binding partners, or by attaching a recruitable element to one of the binding partners or its associated solid support. Other advantages, novel features, and objects of the application will become apparent from the following detailed description of the invention when considered in conjunction with the accompanying drawings, which are schematic and which are not intended to be drawn to scale. In the figures, each identical or nearly identical component that is illustrated in various figures is represented by a single numeral. For purposes of clarity, not every component is labeled in every figure, nor is every component of each embodiment of the application shown where illustration is not necessary to allow those of ordinary skill in the art to understand the application.

In a first aspect of the invention, there is provided a method comprising: exposing a biological or chemical agent, which is linked to a non-colloidal structure, to a binding partner of the biological or chemical agent, which is non-covalently linked to a colloid particle via a metal binding tag/metal/chelate linkage; allowing the colloid particle to fasten to the non-colloidal structure *via* specific interaction between the biological or chemical agent and the binding partner; and determining fastening of the colloid particle to the non-colloidal structure; wherein the colloid particle is derivatized with a self-assembled monolayer.

In a second aspect of the invention, there is provided a composition comprising: a biological or chemical agent, linked to a non-colloidal structure; and a binding partner of the biological or chemical agent, non-covalently linked to a colloid particle via a metal binding tag/metal/chelate linkage; wherein the colloid particle is capable of fastening to the non-colloidal structure *via* specific interaction between the biological or chemical agent and the binding partner; wherein the colloid particle is derivatized with a self-assembled monolayer.

### Brief Description of the Drawings

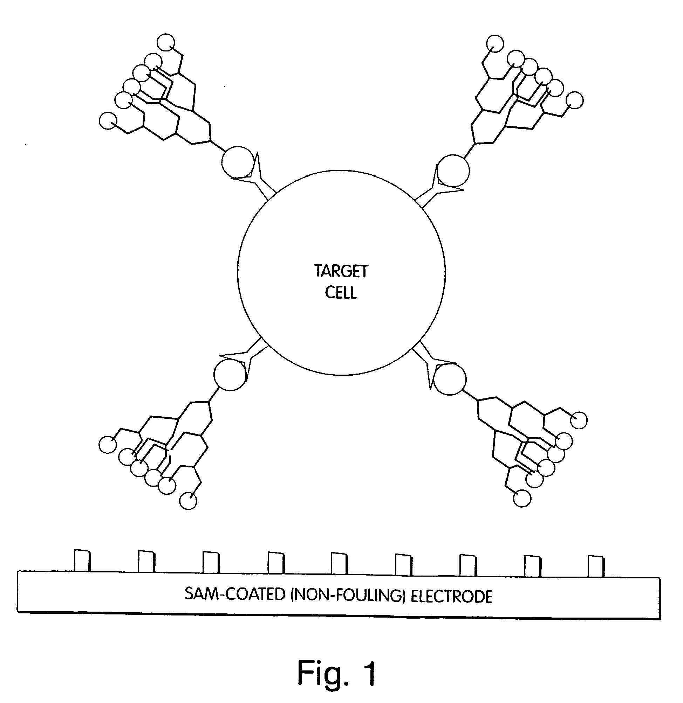

Figure 1 depicts ligands attached to a metal-containing compound, such as ferrocene, via dendrimers or polymers interacting with their cognate cell-surface receptors on intact cells.

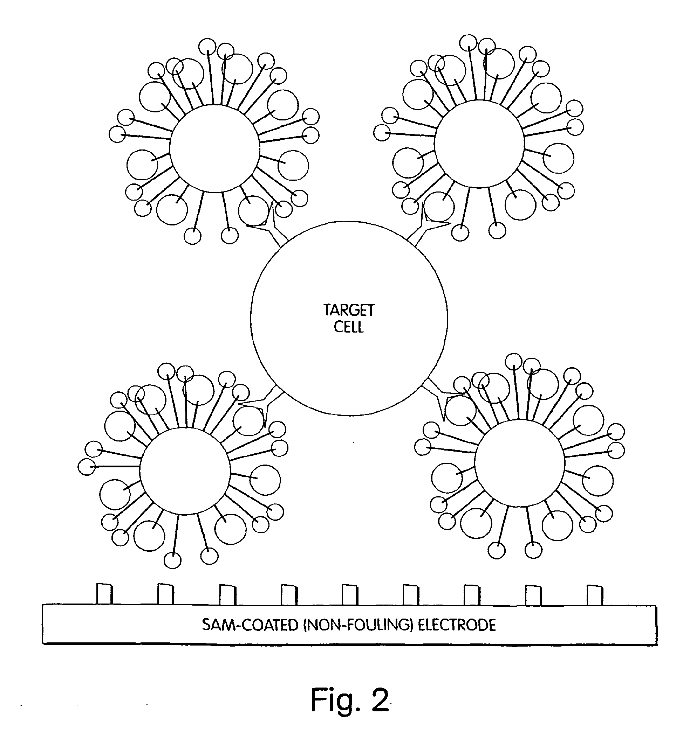

Figure 2 depicts ligands and metal-containing compounds such as ferrocene attached to particles interacting with their cognate cell surface receptors on intact cells.
Figure 3 shows proteins attached to self-assembled monolayers on colloids that also present redox-active metals for electronic or electrochemical signaling. Cells bearing target receptors are incubated with colloids bearing a cognate ligand for the receptor of interest then electrophoretically or magnetically attracted to the sensing electrode. Note that the figure is not drawn to scale and the colloids can be made in a variety of sizes, ranging from 20 gold atoms to hundreds of nanometers in diameter.
Figure 4 shows cells bearing a tumor marker MUC-1 that are incubated with electronic signaling colloids that present the MUC-1 specific antibody, DF-3. The antibody is attached to the colloid via a His-tagged protein G that is bound to NTA/Ni(II) groups that were incorporated into a self-assembled monolayer on the colloid.
Figure 5 shows one embodiment of an assay that can be used to screen drug libraries for the ability of their individual members to disrupt interactions with cell-surface proteins.
Figure 6 shows that a drug screening assay can be readily multiplexed by interfacing microwells (can be disposable) with a microelectrode array and robotics. Analysis of arrays of electrodes can be performed using a modified electrochemical analyzer capable of concurrent analysis of a plurality of electrodes. The electrodes can be configured so as to be inserted (see arrow) into the microwells.
Figure 7 shows that drug candidates can be synthesized on, or covalently attached to, particles that also bear electro-active signaling moieties. Drug candidates attached to signaling particles can be incubated with cells presenting the receptor of interest and control cells that did not express the receptor of interest. A drug-target interaction in this assay will result in a gain of signal.
Figure 8 shows a tissue specimen that is attached to a flexible, semi-permeable support via interaction with peptides containing RGD motifs. The specimen is then incubated with electro-active signaling colloids that bear a ligand for a cell-surface receptor of interest. The specimen is rinsed, then interfaced with a microelectrode array. The electrode array can be made such that the electrode dimensions are comparable to cell dimensions. The specimen is then characterized by ACV, which can then be correlated to histopathology.

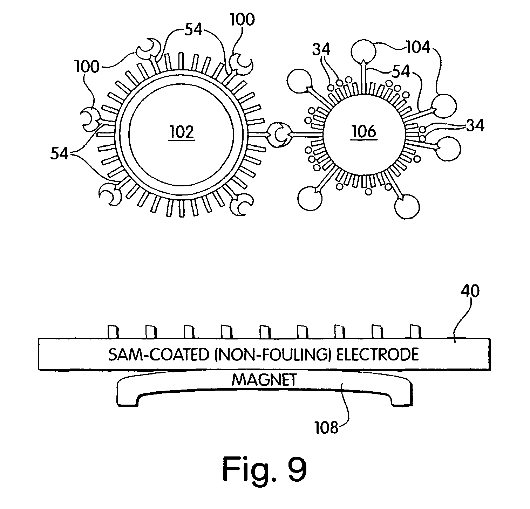

Figure 9 shows a protein-protein interaction assay that is based on universal recruitment and signaling particles that can be easily multiplexed. Self-assembled monolayers bearing NTA/Ni(II) ligands that selectively capture His-tagged proteins are formed on signaling colloids and gold-coated magnetic beads. Each particle type is then briefly incubated with a different His-tagged protein. A magnetic field then attracts the magnetic bead to the sensing electrode. A signal is only transduced when the electro-active labels (which can be redox-active moieties) on the colloid are brought to the electrode through an interaction between a first protein and a second protein. Note that non-His-tagged proteins can also be attached to universal particles that bear self-assembled monolayers presenting carboxy-terminated thiols.

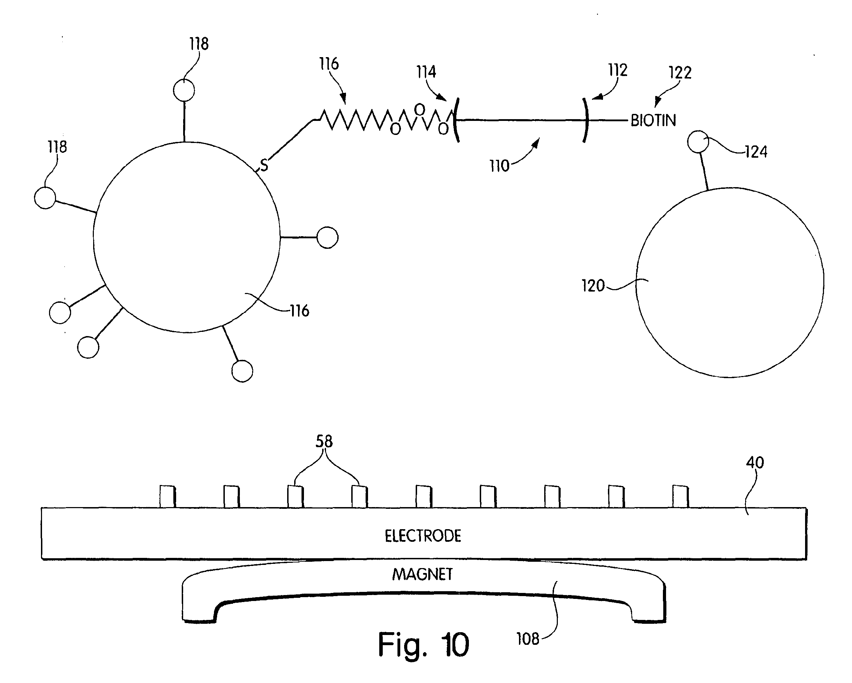

Figure 10 shows electro-active signaling colloids derivatized with thiols bearing enzyme cleavage sites (ECS) and terminated with biotin. Magnetic beads are derivatized with streptavidin, then the magnetic beads and signaling colloids are incubated with the enzyme of interest and drug candidates. If a drug candidate inhibited the action of the enzyme of interest then the site will not be cleaved and the signaling colloid will remain attached to the magnetic bead via the biotinstreptavidin interaction. When the resulting complexes are magnetically attracted to a sensing electrode, a current peak will result, making the assay a gain of signal detection assay.

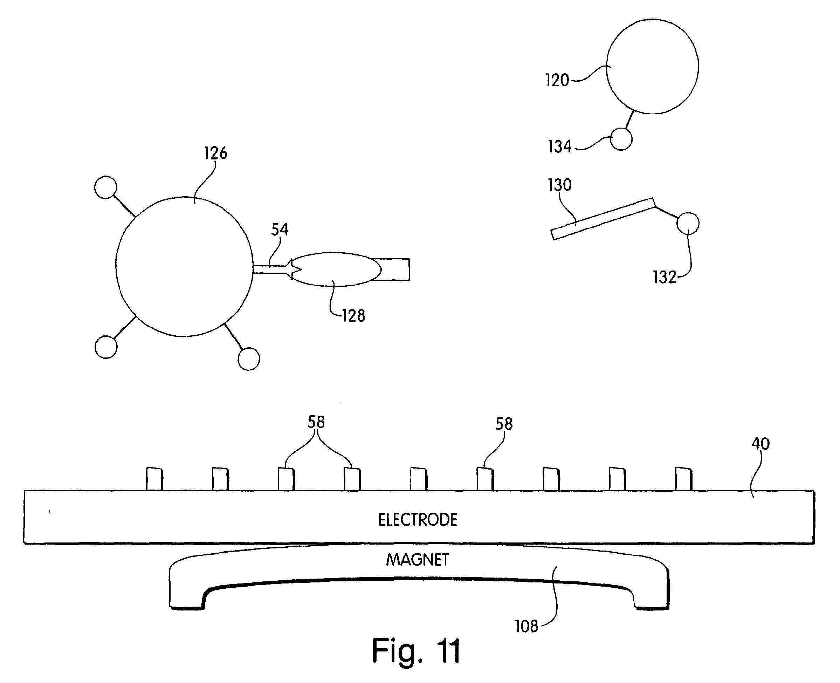

Figure 11 shows a strategy for screening for drugs that modulate enzyme activity. In this scheme, a protein that is a target of enzyme modification is attached to a colloid that also bears an electro-active moiety. The chemical group that the enzyme adds to the target protein has been labeled with biotin. When the enzyme functions properly, the biotin-labeled compound is added to the protein on the signaling colloid. This resulting complex is then connected to a streptavidin-coated magnetic bead. The complex is then electromagnetically attracted to a sensing electrode. Drug candidates can be added to the assay. A loss of signal compared to controls would indicate that the drug candidate inhibited the enzyme's activity while a gain of signal would indicate that a drug enhanced the enzyme's activity.
Figure 12 shows the chemical structures of the farnesyl group, farnesyl pyrophosphate, and geranyl-geranyl pyrophosphate.
Figure 13 shows the modification of farnesyl- or geranyl-like moieties with a recognition group such as biotin.
Figure 14 shows farnesyl pyrophosphate derivatives bearing a biotin moiety. The farnesyl protein transferase (FPT) enzyme catalyzes the addition of the farnesyl derivative to RAS protein variants. The activity of the enzyme, or the ability of the drug candidate to modify enzyme activity can be quantitated by measuring current output when magnetic beads with streptavidin are used to recruit the complexes to the sensing electrode.
Figure 15 shows that drug candidates can be assessed for their ability to modulate enzyme activity. Each spatially addressable microwell contains a RAS protein (wild type or variant), the FPT enzyme, the biotin-modified addition group (farnesyl derivatives), magnetic beads bearing streptavidin, and plus or minus drug candidates. Electrodes can then be analyzed by a variety of electronic and electrochemical techniques, including alternating current voltammetry (ACV).

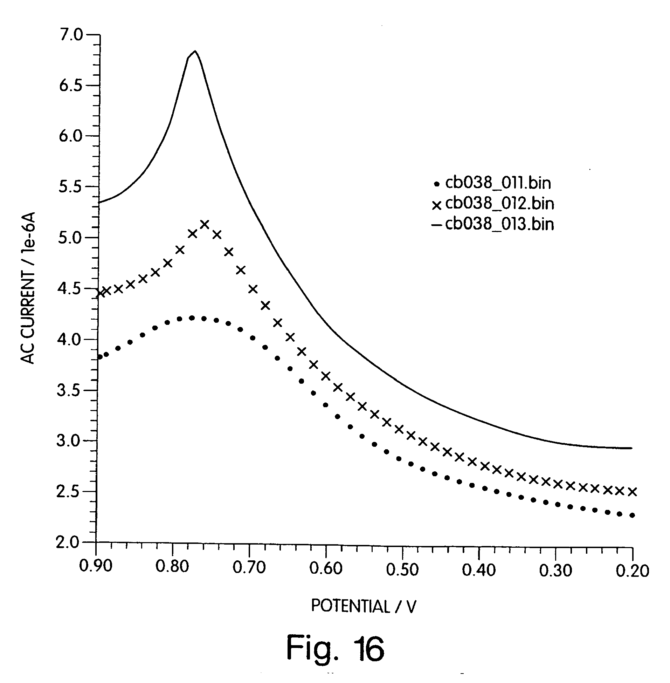

Figure 16 shows a plot of an ACV analysis of the interaction of an RGD-containing ligand with cells grown on collagen-coated electrodes.
Figure 17 shows a plot of an ACV analysis of the interaction of an (negative) control ligand with cells grown on collagen-coated electrodes.
Figure 18 shows a plot of an ACV analysis of cells grown on a metal support comprising only a single type of conductive molecule.
Figure 19 shows a plot of an ACV analysis of the interaction of an RGD-containing ligand with cells grown on a metal support comprising a heterogeneous monolayer of insulating and conducting molecules.
Figure 20 is a photocopy of a photograph of colloid-decorated beads, specifically, colloids linked to beads via protein/protein interaction.
Figure 21 is a photocopy of a photograph of the negative control of the experiment shown in Figure 20.
Figure 22 shows a plot demonstrating the increase in conductivity of the monolayer as the percent of conducting molecules (in a lawn of insulating species) is increased.
Figure 23 shows a plot of an ACV analysis of the conductivity of a monolayer of conductive self-assembled monolayer containing a two-unit molecular wire poly (ethynylphenyl) thiol, MF1 shown against a negative control of a non-conductive self-assembled monolayer, as measured with a solution of ferrocene.
Figure 24 shows a plot of an ACV analysis of the conductivity of a monolayer of the two self-assembled monolayers of Figure 23, as measured against more highly-conductive monolayers, as measured with a solution of ferrocene.
Figure 25 shows ACV analysis of protein/protein interaction as measured by binding of a colloid to a magnetic bead.
Figure 26

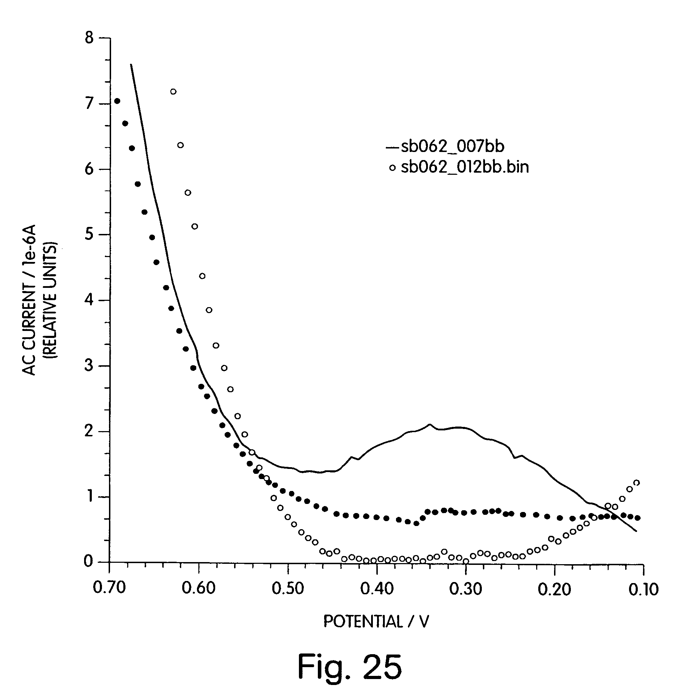

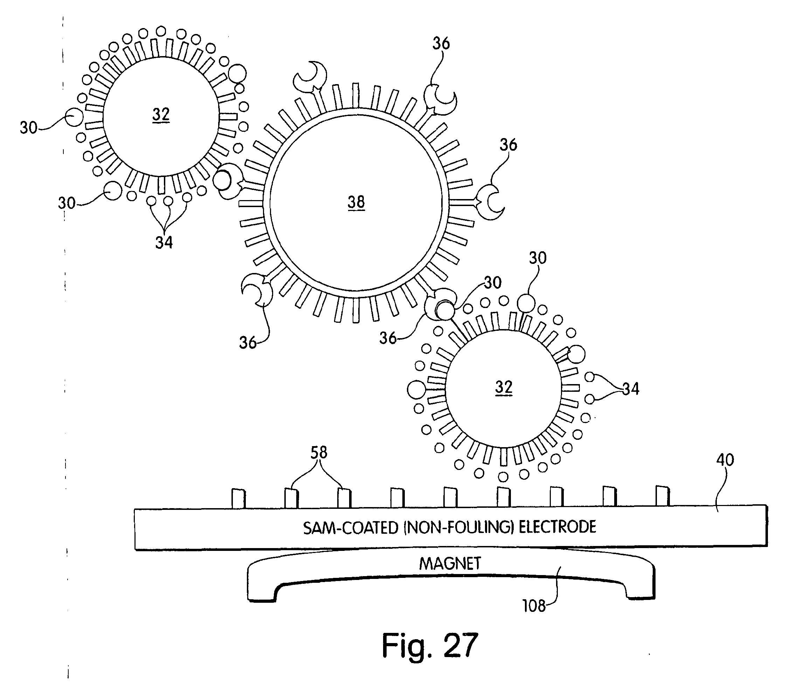

Figure 27 is a schematic illustration of the assay, the plot of which is shown in Figure 25, of protein/protein interaction as measured by linkage of colloid to magnetic bead.

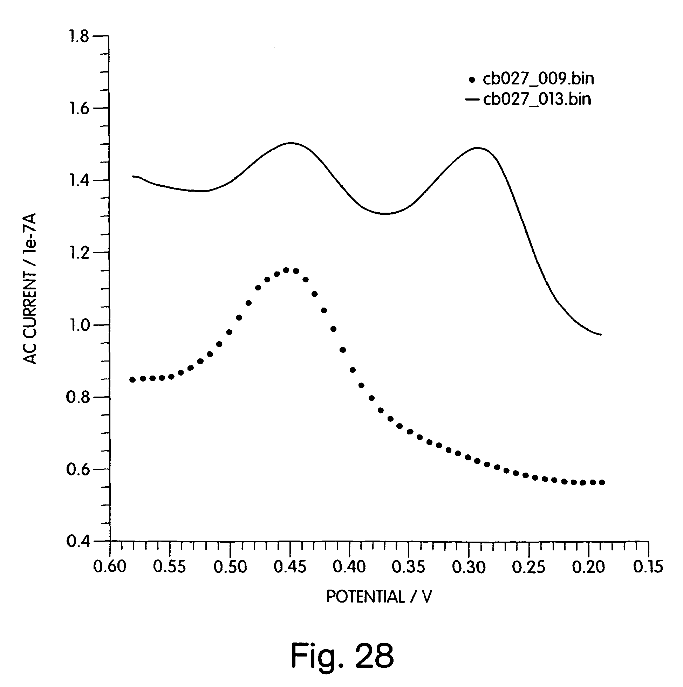

Figure 28 shows a plot of an ACV analysis an electrode containing surface-bound proteins and their affect on ferrocene oxidation potential (solid line), and negative control (dotted line).

### Detailed Description of the Invention

The present application contemplates interaction between chemical or biological agents for analysis, drug screening, or the like. The application includes but is not limited to analyzing and/or inhibiting ligand interactions, including but not limited to ligands on intact cells (growing on an electrode, or in solution or in suspension). The present application contemplates a variety of embodiments, including the use of drug candidates, known or putative ligands, and small molecule drug libraries.

Cells may be grown on electrodes that may or may not be derivatized with self-assembled monolayers (SAMs). Putative ligands (e.g. for a particular cell-surface receptor) are immobilized on a solid support (e.g. gold colloids) along with signaling elements (e.g. electro-active complexes). These derivatized solid supports are incubated with the cells immobilized on a sensing electrode (e.g. metal support). The interaction between the target receptor and the ligand on the solid support (e.g. colloid-bound ligand) tethers the co-immobilized signaling elements near the sensing electrode. The interaction may be detectable as a change in impedance. While not limited to any particular mechanisms, it is believed that, as a potential is applied to the electrode, the nearby redox-active metal complexes go through their characteristic oxidation potential and eject electrons. When an oscillating component is added on top of the voltage ramp, many electrons are ejected by each metal complex and can be detected as current output. A form of this sort of electrochemical analysis is called alternating current voltammetry (ACV).

It is not intended that the present application be limited to the situation wherein the cells are bound directly to a the metal support. It is contemplated that the cells are indirectly bound through the interaction of ligands attached to the metal support. That is to say, cells can also be recruited to a surface by coating that surface with molecules that directly or indirectly bind to cells, by specific or nonspecific interactions. For example, methyl-terminated self-assembled monolayers (SAMs) bind collagen non-specifically, which in turn binds cells non-specifically. Alternatively, peptides that contain an arginine, glycine, aspartate (RGD) motifs, mimic vitronectin and bind specific cell types, like endothelial cells. Similarly, polylysine, positive charge, Kringle domains, integrins, and peptide, or molecular mimics of the same, can be bound to surfaces for the attachment of cells. These ligands can be displayed on a surface by incorporation into SAMs. The ligands themselves need not be directly incorporated into the SAM. SAMs that display a binding partner for an affinity tag attached to the ligand may be used. For example, peptides modified with a histidine tag can be easily attached to SAMs that contain a nitrilo tri-acetic acid (NTA) - nickel thiol. Alternatively, glutathione S-transferase fusion proteins can be attached to a SAM that incorporates glutathione or a derivative thereof. One advantage of using a metal electrode is that many known functional groups can be provided at the terminus of molecules that will form a SAM on the surface.

Cells in solution, for example, can be attracted to a detecting electrode by electrophoresis. Specifically, cell-derived molecules can be bound to a ligand(s) that are attached to a colloid that also displays electro-active compounds such as ferrocene derivatives to aid in the detection of the bound complex. However, the detection element used can be any charged, electro-active species or fluorescent tag that can be easily detected.

The ligand derivatized colloids can be tethered near an electrode that may or may not be modified with a self-assembled monolayer (SAM) and can detect the bound cells by perturbations in the electrode current induced by the binding. Cell-derived proteins can also be bound to a ligand(s) that is attached to a magnetic bead. Alternatively, cell-derived proteins can also be bound to a ligand(s) on an electroactive dendrimer. Additionally, cell-derived proteins can be bound with a ligand(s) that is attached to a polymer that also displays electro-active or redox-active complexes. Cell-derived proteins can also be bound with a ligand(s) that is attached to a conducting polymer that also displays electro-active or redox-active complexes.

In an alternative scheme, the described system can make use of two different ligands. One ligand, which binds a to a constitutively expressed cell surface receptor, can be immobilized on a magnetic bead and used to recruit the cell to a sensing electrode. A second ligand which recognizes the cell surface receptor of interest is attached to a signaling colloid.

In another alternative scheme, the described system can make use of two distinguishable electro-active complexes, such as two ferrocene derivatives that oxidize at different potentials. The first ferrocene derivative can be directly or indirectly attached to a ligand for the cell-surface receptor of interest. A second ferrocene derivative is directly or indirectly attached to a second ligand that binds to a constitutively expressed cell surface receptor. In this way, the ratio of the two signals can be used to calibrate the level of induction of a cell-derived molecule (e.g. protein) that is induced by a physiological, environmental, or disease-associated change in expression. This comparison of expression ratios can also be done by having both ligands carry the same metal complex but incubating separate aliquots of the sample on separate electrode pads.

The present application contemplates the attachment of ligands (including but not limited to putative drug candidates) to a surface that can be particle-like and interact them with cell-surface proteins that can be attached to supports or left intact on cells in an effort to identify binding partners, determine their presence or absence, and quantify their levels. Specifically, intact cells that present cell surface receptors can be used as the first binding partner. Known or putative ligands attached directly or indirectly to electro-active complexes act as the second binding partner. However, the detectable moiety attached directly or indirectly to these ligands can be any convenient charged, electro-active, or fluorescent tag. Alternatively, the first binding partner can be metal binding tagged species such as histidine-tagged (His- tagged) proteins, peptides, small molecules or DNA which are bound to colloids bearing complexes of a chelate such as nitrilotriacetic acid (NTA) coordinating a metal, such as nickel. The colloids may or may not be derivatized with self-assembled monolayers. The second binding partner, which also acts as the detection partner, can be a colloid, a gold surface, or a gold-coated magnetic bead that may also be modified with a SAM.

The first binding partner can also be a magnetic bead that is gold-coated so that an alkyl thiol SAM can be formed on it and capture a histidine-tagged soluble fragment of the cell derived protein. In this case, the second binding partner should have its metal-containing alkyl thiol SAMs on a non-magnetic solid to avoid false positives. Thus the second binding partner can be a colloid, liposome, or polystyrene bead that presents ligands and electro-active complexes on the same surface.

One general technique of the application involves using magnetic beads to recruit an electronic signaling entity to the surface of an electrode indicating capture of a binding moiety (a biological or chemical agent) by a binding partner. Referring to Figure 27, a first species 30 is attached to a colloidal particle 32 that also has a signaling capability carries a signaling entity such as the metallocene ferrocene, 34. A second species 36, which is suspected of being a binding partner of 30 is attached to a magnetic particle 38 that cannot signal, but can be magnetically attracted to an electrode 40. In one particularly useful technique for species 30 and a second species 36 are proteins, thus the application finds particular in the field of proteomics. The technique is a example of an aspect of the application involving allowing a colloid particle 32 the ability to fasten to non-colloidal structure, magnetic bead, 38. Determining fastening of the colloid particle to the non-colloidal structure is carried out by drawing the magnetic bead to the electrode and determining whether the colloid particle is also proximate the electrode, or unattached. Specifically, the two components are incubated together in solution and resultant complexes are magnetically attracted to the electrode. Electrodes are then analyzed by Alternating Current Voltammetry (ACV). (Laviron E: J Electro Anal Chem., 1979, 105: 35). Current is plotted, in real time, as a function of voltage. If the electronic signaling moiety on the first component is brought very close to the electrode, a distinctive current *peak* will occur at a characteristic potential. If the two proteins interact with each other, then when the magnetic particle is attracted to the sensing electrode, it will also carry the colloidal particle, with the signaling capability, with it. Because the first component is a small gold colloid, it will remain in suspension unless it is specifically recruited to the sensing electrode via an interaction with the first species on the colloidal particle and the second species attached to the magnetic particle. An electronic signal will only be generated if the signaling particle has been connected to the magnetic particle through an interaction between the first species and the second species. The colloid particle may comprise an auxiliary signaling entity, exemplified by ferrocene. Alternatively, the colloid particle is itself a signaling entity and no auxiliary entity is required.

This strategy can be used to rapidly screen for protein-protein interactions in array format. When searching for potential therapeutic targets, researchers often need to identify a binding partner for a particular protein. The known protein can be attached to a magnetic bead. This can be accomplished by direct chemical coupling of the known protein to commercially available magnetic beads that present chemically functional groups, by attaching to a protein A-coated magnetic bead via a cognate antibody, or by attaching a histidine-tagged version of the known protein to a gold-coated magnetic bead that has been derivatized with an NTA-SAM (see, for example, U.S. Patent No. 5,620,850). Potential binding partners can be expressed as histidine-tagged proteins then separately attached to NTA-SAM-coated colloids that also present a ferrocene moiety. Each well of a multi-well array, interfaced with an electrode array, will contain the known protein on a magnetic bead. Distinct putative binding partners attached to signaling colloids can be separately added to each well. Resultant complexes can then be magnetically attracted to the electrodes to determined which of the candidate proteins, bound the known protein. The known species need not necessarily be a protein.

Alternatively, biological or chemical agent 30 is a drug candidate and its binding partner (potential binding partner) 36 is a target of a drug candidate or vice versa. Specifically, drug candidates can be attached to the magnetic particles and the known target species attached to signaling colloids, to identify drug candidates that bind to a medically relevant target species. Or, species 30 and 36 can be a target and ligand to the target, respectively, and the technique can involve bringing the colloid 32 and magnetic bead 38 into proximity to allow fastening of 30 to 36 in the presence of a candidate drug for interruption of binding of the ligand to the target. As will be seen below, this technique can be applied to interaction of a colloid with a variety of non-colloidal structures.

Advantages of this technology over existing methods such as ELISA, fluorescent labeling and SPR include: In this system, there is no need for protein labeling; the protein is attached to a labeled component. Gold colloids can be pre-labeled with both: a) a signaling moiety; and b) a functional group for protein attachment. Self-assembled monolayers that present both NTA/Ni²⁺, to capture histidine-tagged proteins, and a ferrocene derivative, for electronic signaling, can be formed on the colloids. SAMs that incorporate carboxylic acid groups, for the chemical coupling (standard EDC/NHS chemistry) of unmodified proteins, can also be used. The technology is modular. Virtually any biological species can be co-immobilized on colloids with a signaling entity. The technology enables cost-effective multiplexing as it can readily be multiplexed on microelectrode arrays.

Liposomes can also be used as the first binding partner 30. As above, the signaling protein itself can be incorporated into the liposome or alkylthiol-containing lipids can be incorporated into the liposomes in order to capture His-tagged soluble receptors after liposome formation. The second binding partner 36 can be a liposome that either had metal-containing compound linked to alkyl thiol chains or lipids incorporated into it. In addition, the liposomes can contain photoactivatable groups that allow for UV-activated cross linking after liposome formation in order to give increased stability.

Tissue slices can also be used to detect cell-derived proteins. The tissue slice is adhered to a dialysis membrane and then incubated with ligands attached to colloids, dendrimers or polymers that have metal containing compounds that bind to cell-derived proteins. The tissue slice is then flipped over and the side having tissue adhered to it is brought near the electrode where it is electronically or electrochemically analyzed. Alternatively, the tissue slice can be adhered to a dialysis membrane via interaction to a binding partner attached to the dialysis membrane or to the electrode via interactions with binding ligands in the SAM-coated electrode. The tissue slice can also be tethered to the dialysis membrane by embedding the binding ligand in a specific pattern in the dialysis membrane.

One can also use this system to screen for drugs that inhibit the upregulation of cell-derived proteins that are involved in various pathological conditions. Once a binding partner (which can include a drug, antibody, or protein/peptide ligand for the cell derived protein) has been identified, the binding partner can be attached to a detection moiety to quantitate the expression level of the cell-derived protein in response to a disease state or a therapy. This can be any assay that tests for the indirect affects of drug candidates on the expression and translocation of cell-derived proteins both to the cell-surface and intracellular compartments.

Identification of drug candidates can also be accomplished by using a competitive inhibition assay. Specifically, a drug candidate free in solution can be separately incubated with the composition. Competitive inhibition to the target cell-derived protein occurring by drug binding to receptor or ligand can be observed as a time or dosage dependent loss of detection signal.

Doing this requires a method of directly or indirectly attaching a signaling moiety to a known ligand and then provided a method of attaching or recruiting the complex binding partners to a sensing surface. The former is described above while the latter is described below.

The sensing surface used can take various forms. However, for illustration purposes, methods for recruiting the binding partner complex are described that use an electrode that is modified with a conducting SAM. A conducting SAM is a layer of molecules attached to a metal surface that allows the conduction of electrons at a rate that is higher than a metal uniformly coated with an insulating species such as saturated alkyl thiolates. A preferred pathway for electron conduction can be provided by a monolayer into which molecular wires (polymers of aromatic ring compounds) have been incorporated. A variety of molecules can be used for this purpose, including but not limited to poly (ethynylphenyl thiol) (i.e. C₁₆H₁₀S), referred to herein as MF1.:

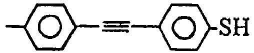

"Molecular wires" as used herein, means wires that enhance the ability for a fluid encountering a SAM-coded electrode to communicate electrically with the electrode. This includes conductive molecules or, as mentioned above and exemplified more fully below, molecules that can cause defects in the SAM allowing fluid contact with the electrode. A nonlimiting list of additional molecular wires includes 2-mercaptopyridine, 2-mercaptobenzothiazole, dithiothreitol, 1, 2-benzenedithiol, 1, 2-benzenedimethanethiol, benzene-ethanethiol, and 2-mercaptoethylether. Conductivity of a monolayer can also be enhanced by the addition of molecules that promote conductivity in the plane of the electrode. Conducting SAMs can be composed of, but are not limited to: 1) poly (ethynylphenyl) chains terminated with a sulfur; 2) an alkyl thiol terminated with a benzene ring; 3) an alkyl thiol terminated with a DNA base; 4) any sulfur terminated species that packs poorly into a monolayer; 5) all of the above plus or minus alkyl thiol spacer molecules terminated with either ethylene glycol units or methyl groups to inhibit non specific adsorption. Thiols are described because of their affinity for gold in ready formation of a SAM. Other molecules can be substituted for thiols as known in the art from U.S. Patent No. 5,620,820, and other references. Downstream of the interactive SAM is a charge reader (e.g. ammeter, charge counter) or an optical reader (e.g. surface plasmon resonance detector, or fluorescence reader).

When known ligands on signaling colloids interact with their cognate cell derived proteins, drug candidates can be added in solution and differential effects monitored. Cells can be recruited to an electrode coated with a conducting SAM that also presents molecules such as alkyl thiols, that are terminated with head groups that directly or indirectly bind to cells (e.g. methyl groups, poly K, positive charge, RGD sequences, Kringle motifs, integrins, and peptide mimics of the same). Alternatively differential interactions can be quantified by having cell surface receptors that are interacting with their cognate ligands presented on a conducting SAM that are linked to the SAM via an alkyl thiol or a metal binding tag. In this situation, the electronic signal is generated by a ligand to a constitutively expressed receptor. Under flow force the electronic signal should be proportional to the number of cell surface receptors interacting with SAM-immobilized ligands. The rate of cell mobility as a function of flow rate indicates the density of receptors to cell adhesion molecules on the cell surface.

Cells should be attracted to the sensing electrode due to the fact that they are negatively charged if a slight positive bias to the AC voltage ramp is applied. Adding additional negatively charged groups to the dendrimers or polymers to which the ligands are attached further facilitates recruitment to the sensing surface. Those of ordinary skill in the art can readily fasten a plurality of signalling entities to a dendrimer or polymer. Alternatively, attaching the recognition ligands via metal binding tags facilitates their binding (and consequently the binding of the associated cell) to alkyl thiol NTA. SAMs having metal containing compounds, such as ferrocene, on a gold coated magnetic bead allow the use of an electromagnetic field or a stationary magnet to recruit the complex to the sensing surface electrode. Also sensing electrodes that incorporate ligands, either directly or through an alkyl thiol-histindine tag, can be used to attract cells via specific interactions, such as with cell adhesion molecules or non specific interactions.

However, recruitment to the detection system can be performed also simply by using gravity if the complex sediments by gravity as a result of one of the binding partners being denser than the analysis solution. Mechanical mixing can be performed during the incubation stage to avoid premature sedimentation.

In a variation that uses the electrode as a cell derived protein generator, a point on the electrode pad can be used to produce a voltage spike that lyses the cell. The contents of the cell are then incubated with signaling ligands and interactions are detected.

Additionally cell binding molecules can be patterned in a given topology along the bottom of a flow channel using SAMs in order to line the channel with conducting molecules and enhance the detection abilities of the system. Thus, the SAM can present the binding moiety in a pattern that alternates cell binding capability with sensing capability (optical or electronic). As an example, stripes of methyl terminated alkyl thiols are alternated with conducting poly pyrrole groups in the SAM. The topology of the SAM can also be manipulated such that the cell lies in a furrow so that receptors along the sides of the cell can be assessed. Forming stripes of alternating functionality SAMs such as these can be carried out as described in U.S. Patent No. 5,512,131 and International Patent Publication No. WO 96/29629.

These complexes can be electronically detected. The preferred detection method is an electrochemical technique called alternating current voltammetry (ACV). This detection method can be supplemented by the use of additional analysis techniques such as higher order harmonic analysis. These complexes many also be able to be detected by optical means such as SPR. In using the latter technique, large shifts in optical properties are caused by recruitment of the complexes to the sensing surface. Also, when ligands are bound to hydrogels their interaction with binding partners can cause a change in density of the hydrogel that can be optically detected.

These methods can be used to detect cell surface markers on free cells that are derived from a variety of sources including blood and needle biopsies. They can also be used to screen for drugs that inhibit specific interactions, such as those known or suspected to promote angiogenesis and cancer metastasis in a direct *in vitro* binding assay. Cell surface receptors of particular interest are those that have been correlated with tumorigenesis since blocking these receptors can kill the tumor. Perhaps of even more widespread interest is the over-expression of cell surface receptors that bind to adhesion molecules of other cells. These cell interactions appear to be the mediators of metastasis and angiogenesis.

In addition, these methods can also be used to screen for drugs that inhibit a cell phenotype known to be characteristic to invasiveness or metastasis without a priori knowledge of the specific receptor-adhesion molecule interactions involved. Screening for drugs that mimic a physiologically relevant interaction can also be accomplished using this system whether by adding a drug candidate free in solution and looking for a loss of detection signal, or by adding a known ligand free in solution with the drug candidate on the solid support and looking for the occurrence of a detection signal.

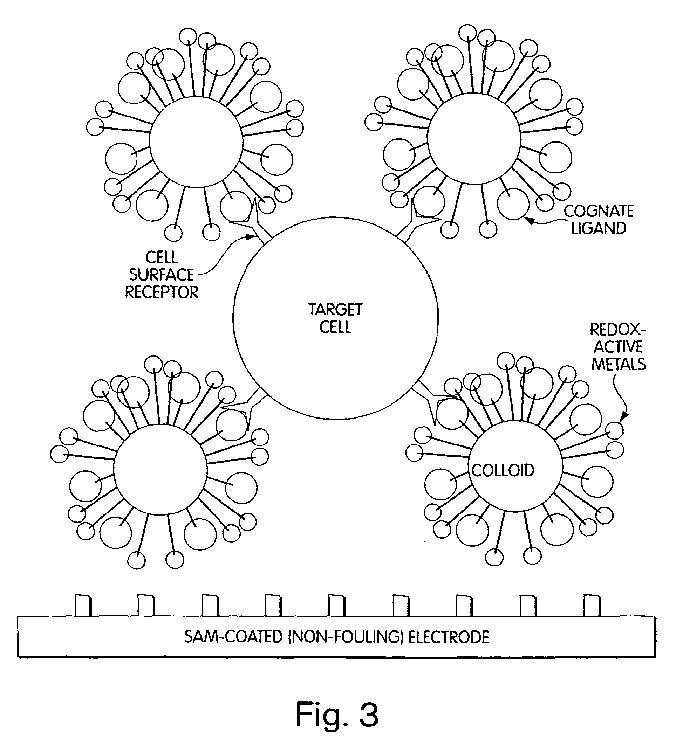

One can detect and quantitate cell surface proteins as follows: Histidine-tagged ligands that recognize cell surface receptors are attached to colloids that bear SAMs presenting both NTA (to capture His-tagged proteins) and ferrocene moieties (for electronic signaling). These biospecific, electronic signaling colloids are then incubated with cells presenting target receptors. Cells are then allowed to sediment, adhere, or be attracted onto to a SAM-coated electrode and analyzed by ACV (Figure 3). A current peak, at the ferrocene moiety's characteristic oxidation potential, will result if ligands immobilized on signaling colloids bound to their cognate receptors on the cell surface. Antibodies that recognize the cell surface receptor can be attached to NTA-ferrocene bearing colloids that have first been bound with His-tagged protein A or G. Alternatively, an antibody can be attached directly to a colloid via a metal binding tag/metal/chelate linkage, where the metal binding tag is linked to the antibody. Techniques for linking a histidine tag to an antibody can be found in "Construction of the single-chain Fv from 196-14 antibody toward ovarian cancer-associated antigen CA125" Hashimoto, Y., Tanigawa, K., Nakashima, M., Sonoda, K., Ueda, T., Watanabe, T., and Imoto, T.: 1999, Biological and Pharmaceutical Bulletin, Vol 22: (10) 1068-1072.; "Human antibodies "Human antibodies with sub-nanomolar affinities isolated from a large non-immunized phage display library", Vaughan, T.J., Williams, A.J., Pritchard, K., Osbourn, J.K., Pope, A.R., Earnshaw, J.C. et al. 1996, Nature Biotechnology Vol 14 (3) pgs 267.; "Expression and purification of single chain anti-HBx antibody in E. coli" Zhou G, lui KD, Sun HC, Chen YH, Tang ZY, and Schroder CH, 1997, vol. 123(11-12) pgs 609-13.

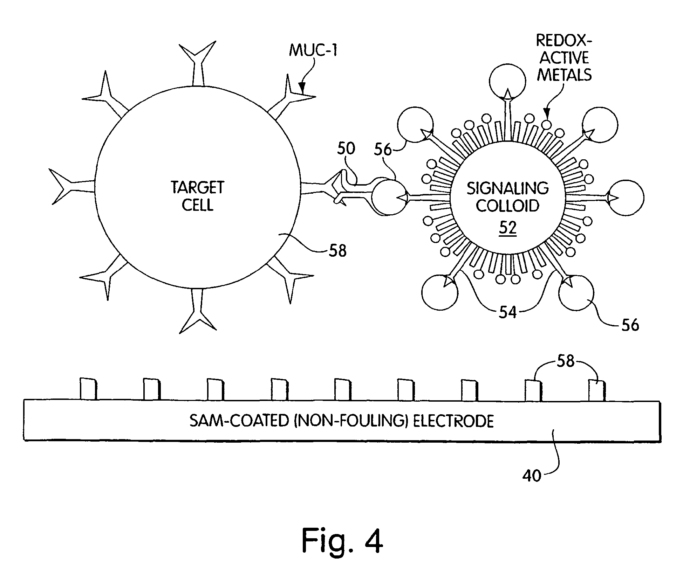

As one example of a technique of the application, with reference to Figure 4, an example of a useful technique involving fastening of a colloid particle to a cell is described. The tumor marker, MUC-1, is aberrantly expressed on neoplastic cells. The human tissue culture breast carcinoma cell line, MCF-7, available from the ATCC, overexpresses MUC-1. Antibody 50, DF3 or and DF3-p, available from the Dana-Farber Cancer Institute, is attached to electronic signaling colloids 52 (bearing NTA-SAMs 54) via a histidine-tagged protein G 56. Target cells 58 re incubated with the antibody-bearing signaling colloids 52, then electrophoresed to an electrode 40 coated with a SAM containing molecular wires 58 and analyzed by ACV. The SAM on electrode 40 includes molecular wires 58 admixed within more conventional, tight-packing SAM-forming species. For simplicity of illustration in all figures, only molecular wires 58 are shown schematically. A current peak results if the antibody-bearing signaling colloids are incubated with cells bearing MUC-160. Alternatively, the putative cognate ligand for MUC-1, I-CAM can be His-tagged and attached to signaling colloids that also bear NTA groups.

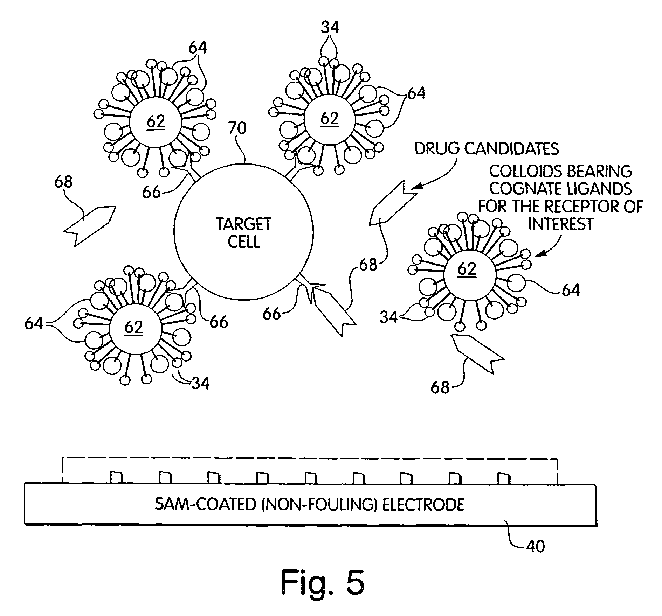

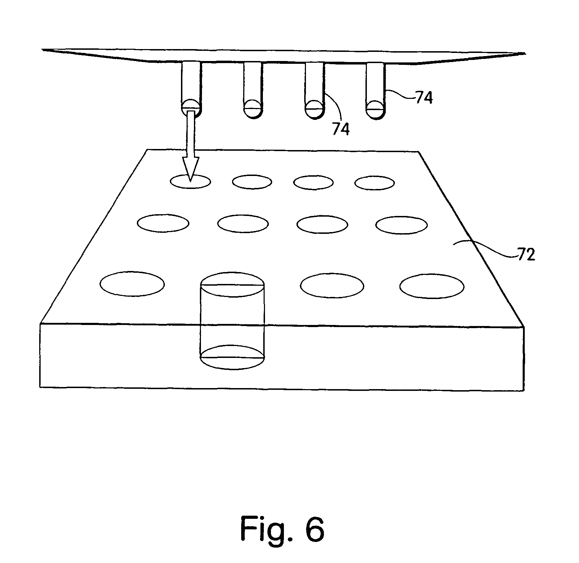

Another assay is shown in Figure 5. Drug libraries can be screened for their ability to disrupt specific interactions with cell surface proteins, such as the MUC-1/1-CAM interaction. I-CAM 64 is bound to signaling colloids 62 as described above, then incubated with cells 70 presenting MUC-166 and control cells. Drug candidates 68 are added to the solution within which the cells and colloids are suspended, then the cells adhere to the electrode and analyzed by ACV. A loss of signal indicates an interaction with a drug candidate. Figure 6 illustrates how this scheme can readily be multiplexed to simultaneously screen thousands of drug candidates using arrays of disposable microwells 72 with interfacable arrays of microelectrodes 74. Each well contains an assay as shown in Figure 5 or another assay.

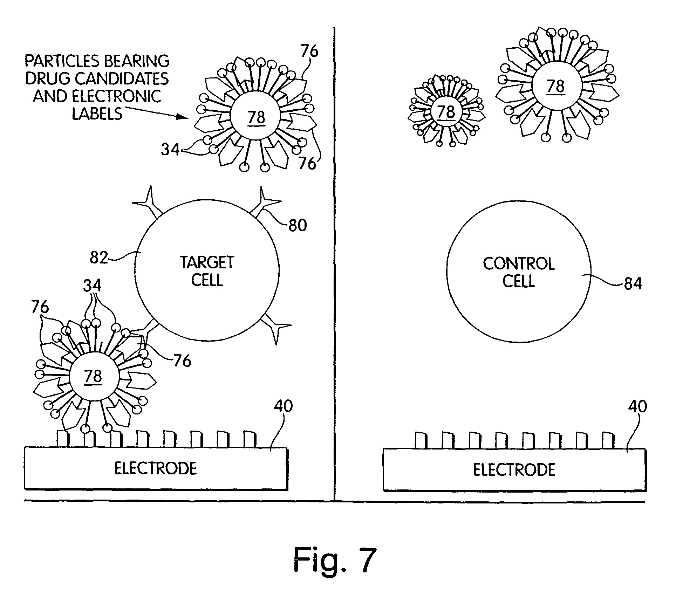

Referring now to Figure 7, an arrangement is shown in which for a gain of signal assay, or to screen for drugs to bind cell surface receptors 80 for which the cognate ligand is not known, small molecule drug libraries can be synthesized on, or covalently attached to, colloids or particles 78 that also bear electronic signaling moieties 34. Drug candidates 76 attached to signaling particles can be incubated with cells 82 presenting the receptor 80 of interest, or control cells 84. A drug-target interaction in this assay will result in a gain of signal (Figure 7).

The cell surface receptor, αVβ3, has been implicated in promoting angiogenesis through an interaction with a cell adhesion molecule vitronectin. Human umbilical veinous endothelial cells (HUVEC) that present αVβ3 cell surface receptors are commercially available from Clonetics. To screen for drugs that inhibit the action of αVβ3, His-tagged peptides that present RGD-containing sequences, derived from vitronectin, are attached to colloids that bear SAMs presenting both NTA groups and ferrocene moieties. The biospecific signaling colloids are then incubated with HUVEC cells and drug candidates. The HUVEC cells can be grown on the electrode. However, if the cells are in solution or suspension they can be electrophoresed or magnetically attracted to a sensing electrode and analyzed by ACV. A current peak occurs when biospecific colloids are incubated with HUVEC cells, rather than control cells. If a drug candidate interferes with the αVβ3-RGD sequence interaction, a loss of signal results.

Alternatively, drug candidates can be synthesized on, or attached to, beads, colloids or supports that also present electronic signaling moieties. These "particles" are incubated with target cells, attracted to a sensing electrode and analyzed by ACV. The attractive field is then reversed and peptides containing RGD sequences are titrated into the solutions. The cells are again electrophoresed to the sensing electrode and re-analyzed. A loss of signal indicates that the drug-cell interaction is specific for the αVβ3 receptor and the IC₅₀ of the RGD peptide can be correlated to a binding affinity for the drug.

Alternatively, a 49 amino acid peptide, echistatin, that also binds to αVβ3, can be His-tagged to replace the RGD-containing peptide in the above-described assay.

Metallocenes are particularly useful as signaling entities for the following reasons. Various ferrocene derivatives and be selected to each oxidize at unique voltage between 100 mV to 800 mV. Each oxidation potential represents a unique label so that proportional to the number of cell surface receptors that were recognized by the signaling colloids.

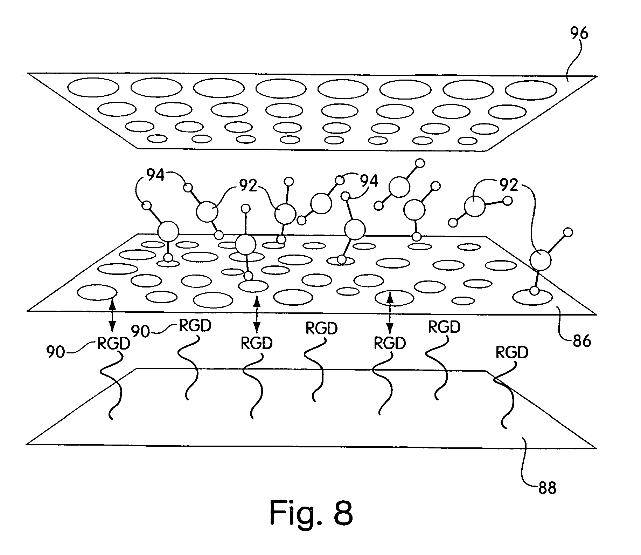

Cell-surface molecules can be detected on cells in suspension or embedded in a tissue sample, as shown in Figure 8. Frozen tumor specimens 86 are cryo-sectioned and placed directly onto a flexible, semi-permeable membrane support 88 that has been derivatized with cell-binding groups 90 such as RGD-containing peptides or methyl-terminated groups. The specimen is then incubated with electronic signaling colloids 92 that also present ligands 94 for a cell surface receptor of interest. Unbound colloids are washed away after an incubation period. The support membrane is then placed in physical contact with a microelectrode array 96, having electrode dimensions comparable to cell size, and analyzed by ACV. Each sector of the tissue specimen is analyzed for protein content and expression level, then correlated with histopathology. This capability ensures the relevance of single cell analysis because it enables the researcher to identify protein patterns that are associated specifically with cancer cells and discard random aberrant protein expression. Cells in suspension can be similarly attached to the support membrane.

The biospecific colloids, described above, can also be used to facilitate *in vivo* imaging. The colloids used are gold, which is relatively inert. Gold colloids that have been derivatized to present an antibody or other ligand for a tumor marker can be taken internally or injected. A tumor mass presenting the cognate tumor marker will become covered with colloids, acting as a concentration device. The tumor can then be detected by imaging techniques, such as X-Ray and X-Ray computer tomography (CT), since the effective result can be a tumor mass enshrouded in metal. Individual colloids in solution would be invisible to detection. Tumors that have been so labeled with biospecific colloids can also be detected by MRI (magnetic resonance imaging). Imaging may be enhanced by incorporating paramagnetic metals and other contrast agents, such as Fe, Gd, or Cr. Contrast agents can be attached to the colloids by incorporating metal chelating thiols into the SAMs that are formed on the colloids. Alternatively, SAMs that only present a cognate ligand for the target protein expressed on the tumor, can be formed on particles composed of the contrast agent material. Colloids having an iron or magnetite core can be gold-coated then derivatized with biospecific SAMs. Alternatively, liposome-like particles can be formed from lipid chains terminated in chelating groups such that the metal contrast agents form the "core" and the biospecific ligands are exposed to solvent. Alternatively, the colloids can bear, in addition to the biospecific ligand, moieties that have a specific spectroscopic signature, which can be detected by energy absorption or scattering techniques, including Raman Spectroscopy. The imaging apparatus can be contained outside of the body or introduced into the body on scopes or optical fibers. Alternatively, colloids bearing moieties that transmit radio frequencies can generate a detectable signal if concentrated onto a tumor mass. The signal from individual colloids should be negligible or undetectable compared with the signal generated from a concentration of colloids bound to a tumor mass.

This technology can be developed into a universal protein interaction tool kit that researchers can use for the study of protein-protein or drug-protein interactions that are important for signal transduction and for the identification of potential drug targets. The technology is inexpensive because the electronics of the system uses cheap, off-the-shelf components and the bioelectronic "particles" are self-assembled from universal components that are synthesized off-line. The system may be based on universal signalling colloids and universal recruitment particles. SAMs presenting NTA can be rapidly formed on any gold substrate. The NTA group, when coordinating a nickel atom, specifically captures histidine-tagged proteins. SAMs that present NTA-thiols and tri-ethylene glycol-terminated thiols have been shown to selectively capture histidine-tagged proteins while resisting the non-specific adsorption of others (U.S. Patent No. 5,620,850). This means that surfaces that capture histidine-tagged proteins can be formed on gold-coated magnetic beads or on gold colloids that also present ferrocene-thiol signaling moieties. These pre-formed particles need only be briefly incubated with the desired histidine-tagged protein to create biologically specific recognition particles.

With reference to Figure 9, to determine whether two proteins interact with each other, one will bind the first protein 104 to a colloid 106 presenting both NTA 54 and ferrocene ligands 34 and bind the second protein 100 to a SAM-coated magnetic bead 102 that presents only the NTA ligand 54. The two particle types are mixed together then magnetically recruited to a sensing electrode 40 under which is a magnet 108. An electronic signal will only be generated if the magnetic bead were connected to the signaling colloid through the interaction of the two protein species. Non-histidine-tagged proteins can also be attached to signaling or magnetic (recruitable) particles by forming SAMs on them that incorporate carboxy-terminated thiols. Standard EDC/NHS coupling chemistry can then be used to attach any molecule that presents a primary amine. Because the electronic labeling step has been separated from the protein preparation step, the system can be efficiently multiplexed using universal electronic signaling colloids and magnetic particles. This facilitates the construction of protein interaction databases to help researchers decipher the molecular profiles of cancers.

Interacting protein partners can be incubated with small molecule drug libraries and drug leads identified by detecting a loss of signal. Alternatively, small molecule drug libraries can be purchased on magnetic beads to directly screen for drug candidates that bind to a known, target protein.

The basic technology described herein can also be used to screen for drugs to modulate enzyme activity. Certain enzymes have been identified as being critical to the progression diseases. In some cases a molecule must be cleaved at a specific site for it to become active and one would like to screen for drugs that inhibit the activity of the enzyme that does this. The technology described herein can be used as described below for this purpose:

Useful in carrying out this technique is the following: Edelstein and Distefano have reported that farnesyl pyrophosphate groups modified with photoactivatable cross-linking moieties can be added to peptide motifs derived from RAS by yeast FPT (Edelstein R. and Distefano M. (1997). Photoaffinity labeling of yeast farnesyl protein transferase and enzymatic synthesis of a RAS protein incorporating a photoactive isoprenoid. Biochem. and Biophys. Res. Comm. 235, 377-382). Their objective was to use this assay to show that farnesyl derivatives were recognized by FPT, by cross-linking the two. This finding is consistent with the idea that farnesyl or garenyl moieties can be modified with biotin at the end distal from the pyrophosphates without interfering with enzyme activity or specificity.

With reference to Figure 10, an enzyme cleavage site (ECS) 110 can be attached at one end 112 to a molecule 122 for which there is a known binding partner (BP1). At the other end 114, it can be attached to a molecule 116 that can incorporate into a SAM (HS-R-X), where S is sulfur, R is an molecular species that can incorporate into a SAM, and X is a linker. The resultant molecule can be incorporated into SAMs on colloids 116 along with groups 118 that can deliver an electronic signal (HS-R-XM), where M is a redox-active metal or transition metal (or other group capable of transducing an electronic signal when brought close to an electrode). Magnetic beads or particles 120 can be derivatized to present a molecule 124 (e.g., streptavidin) (BP2) that binds to BP1 122 either directly or indirectly through a simultaneous binding to a mutual target molecule (BP3).

For example, electronic signaling colloids 116 can be derivatized with thiols bearing enzyme cleavage sites (ECS) and terminated with biotin. Magnetic beads can be derivatized with streptavidin. Magnetic beads and signaling colloids can be incubated with the enzyme of interest and drug candidates. If a drug candidate that can moderate, or inhibit the action of the enzyme, then the site (of e.g., a protein or peptide) would not be cleaved, and the signaling colloid would be attached to the magnetic bead through the biotin/streptavidin interaction. When the complexes are magnetically attracted to a sensing electrode, a current peak would result, making the assay a gain of signal detection assay. That is, a protein can be adapted for linkage both to colloid 116 and bead 120 via a SAM-forming species at one end and a binding partner 122 plankable to an immobilized binding partner 124 on bead 120, and cleavage, or moderation thereof, monitored. The protein can be linked to the colloid and bead in a variety of ways including that described or, in addition, the colloid can present a chelate coordinating a metal, the protein can be provided with a metal binding tag. The application is even more generalized, in that any entity adapted for linkage both to the colloid particle 116 and any non-colloidal structure, such as bead 120, can be presented to the colloid and the non-colloidal structure and allowed to link thereto, in the presence both of an enzyme having the ability to cleave the entity and a candidate drug for moderation of activity of the enzyme. The non-colloidal structure can be a bead as illustrated, or an electrode itself.

BP1 can be the same as BP2 and/or BP3. For example, the thiol that bears the ECS and BP1 (biotin) can bind to free streptavidin (BP3) then to biotin (BP2) on the magnetic beads.

Alternatively, one may seek to inhibit or accelerate an enzyme that adds a molecular group to a target molecule, i.e., one can expose a colloid particle and a non-colloidal structure to a substrate for an enzyme adapted for linkage to the non-colloidal structure, a molecular species linkable to the substrate via enzyme activity adapted for linkage to the particle and an enzyme for the substrate. This can be carried out further in the presence of a candidate drug for moderation of the enzyme, and the non-colloidal structure can be a magnetic bead with the non-colloidal structure to a substrate for an enzyme adapted for linkage to the non-colloidal structure, a molecular species linkable to the substrate via enzyme activity adapted for linkage to the particle and an enzyme for the substrate. This can be carried out further in the presence of a candidate drug for moderation of the enzyme, and the non-colloidal structure can be a magnetic bead with the colloid particle carrying an immobilized electroactive entity. Alternatively, the non-colloidal structure can be an electrode surface.

Specifically, with reference to Figure 11, in such a case, electronic signaling colloids 126 are derivatized with the molecule 128 via chelate/metal/metal binding tag linkage, to which the group is to be added. The piece to be added, the addition molecule 130, is terminated with a first binding partner (BP1) 132 (can be biotin). The magnetic bead 12 presents a binding partner of BP1, (BP2) 134 (can be streptavidin). Colloids, magnetic beads, addition molecules, the enzyme of interest and drug candidates are incubated then attracted to an electrode for analysis. A loss of signal, compared to controls, indicates that the drug candidate inhibited the enzyme's activity while a gain of signal indicates a drug enhanced the enzyme's activity.

A wide variety of SAMs can be used in accordance with the application, on a wide variety of surfaces, to present desired species such as binding partners, signaling entities, and the like at a surface of an article such as an electrode, colloid particle, or the like. Those of ordinary skill in the art can select from among a wide variety of surfaces, functional groups, space for moieties, etc. An exemplary description can be found in U.S. Patent No. 5,620,850. This U.S. Patent also describes a variety of metal binding tags that can be used, including nitrilotriacetic acid, 2,2'-bis(salicylideneamino)-6,6'-demethyldiphenyl, and 1,8-bis(a-pyridyl)-3,6-dithiaoctane, or the like.

A variety of non-colloidal structures comprising beads are described above. The beads can comprise polymeric material, agarose, tentagel, and/or magnetic material. Polystyrene beads are quite useful. The function and advantage of these and other embodiments of the present application will be more fully understood from the examples below. The following examples are intended to illustrate the benefits of the present application, but do not exemplify the full scope of the application.

The following examples and experiments illustrate particular embodiments of the present application and are not to be construed as limiting the application to any particular embodiment.

### Examples

Some examples below involve SAM formation, collagen coating, cell growth, colloid formation, and Alternating Current Voltammetry (ACV). For SAM formation, glass microscope slides were sputtered with a layer of Ti followed by a layer of Au. Each electrode was incubated at RT for 0.5 hours with 300 uL of a DMF solution that contained 10% methyl-terminated thiol (HS-(CH2)15 CH3), 40% tri-ethylene glycol-terminated thiol, HS(CH₂)₁₁(CH₂CH2)₃OH, (formula) and 50% DMF-1. 2 ml of 400 uM tri-ethylene glycol-terminated thiol were then added to a scintillation vial containing the chip and the vial was heat cycled in a water bath as follows: 2 minutes @ 55°C; 2 minutes @ 37°C; 1 minute @ 55°C; 2 minutes @ 37°C then RT for 10 min. Electrodes were then dipped in EtOH, then sterile PBS to rinse. They were then placed under the LTV germicidal lights in a biosafety cabinet for I hour to ensure sterility.

For collagen coating, a 200 uL droplet of 0.005 mg/ml collagen in PBS was added to each electrode and incubated at 4°C for 2 hours.

For cell growth, the electrodes were placed in a cell growth flask and a solution of growth media and human endothelial cells (HUVECs), presenting a particular cell surface receptor, αVβ3, was added. The electrodes and cell containing solution were incubated at 37 °C in a C0₂ incubator for 24 hours. Visual inspection with 100X magnification showed that the cells had adhered to the electrodes and showed web-like spreading which is a growth phenotype.

For colloid preparation, 1.5 ml of commercially available gold colloid (Auro Dye) were pelleted by centrifugation in a microfuge on high for 10 minutes. The pellet was resuspended in 100 uL of the storage buffer (sodium citrate and tween-20). 100 uL of a dimethyl formamide (DMF) solution containing 90 uM nitrilo tri-acetic acid (NTA)-thiol, 90 uM ferrocene-thiol, and 500 uM carboxy-terminated thiol. Following a 3-hour incubation in the thiol solution, the colloids were pelleted and the supernatant discarded. They were then incubated in 100 uL of 400 uM tri-ethylene glycol-terminated thiol in DMF for 2 minutes at 55 °C, 2 minutes at 37 °C, 1 minute at 55 °C, 2 minutes at 37 °C, either: a) a peptide designed to bind to the alpha V beta 3 receptor,
HHHHHH(S₄G₁)₃GRGDSGRGDS; or b) an irrelevant peptide, HHHHHH-Glutathione S-Transferase (GST). Peptides containing an RGD motif have been shown to bind to the alpha V beta 3 receptor on endothelial cells. It is thought that RGD motifs in vitronectin (the natural ligand for alpha V beta 3) are responsible for the interaction.

ACV Analysis was performed using a CH Instruments electrochemical analyzer. A three-electrode system was used. A silver vs. silver chloride reference electrode was used with a platinum auxiliary electrode. The derivatized gold-coated chip was used as the working electrode. A 25 mVolt overpotential was applied to the electrode at a frequency of 10 Hz.

### EXAMPLE 1

This example describes an assay to screen for inhibitors of farnesyl protein transferase (FPT) and geranylgeranyl protein transferase (GGPT).

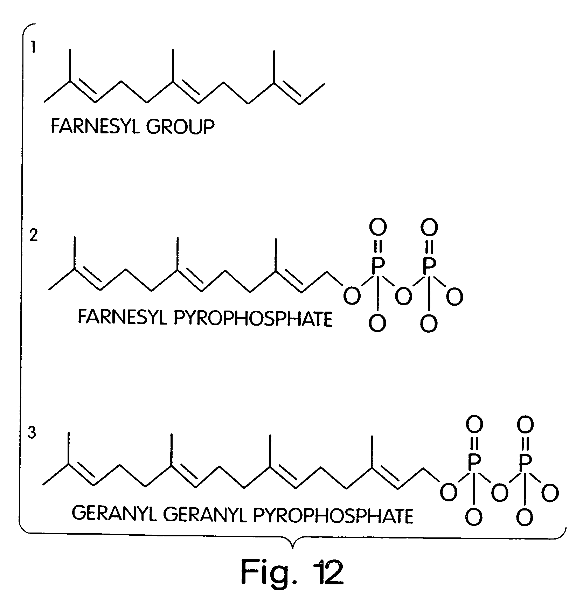

RAS proteins are small GTP-binding proteins that affect signal transduction, differentiation and proliferation. Mutations in RAS, which decrease GTPase activity, have been found in 40% of human cancers and result in constitutive RAS activity (Bos. JL (1989) Cancer Res. 49, 4682-4689). However, for RAS to be active, it must be localized to the cell membrane. Before RAS can attach to the cell membrane, an enzyme, farnesyl protein transferase (FPT), must add a lipid-like group to the C-terminus of RAS, to facilitate membrane attachment (Zhang FL, Kirschmeier P, Carr D, James L, Bond R, Wang L, Patton R, Windsor W, Syto R, Zhang R and Bishop WR. (1997). Characterization of Ha-ras, N-ras, Ki-ras4A and Ki-ras4B as in vitro substrates for farnesyl protein transferase and geranylgeranyl protein transferase type I. J Biol Chem 272:15, 10232-9). FPT transfers a farnesyl group, 1 of Figure 12, from its prenyl donor farnesyl pyrophosphate 2 to the sulfur of cysteine on proteins that contain the C-terminal motif CAAX, where C is cysteine, A is aliphatic and X is methionine or serine. Substrates for the related enzyme geranylgeranyl protein transferase (GGPT) typically have a C-terminal leucine, although the enzyme can add geranyl groups, 3, to RAS proteins which have the CAAX motif, albeit at a rate that is much slower than the farnesylation process catalyzed by FPT. The modulation of these enzymes has become an important area of research for cancer therapeutics, since inhibitors of FPT and GGPT activity abolish Ras's ability to transform cells. Strategies to inhibit FPT and GGPT activity, which are under development, include: 1) the use of short peptide motifs and peptide mimics to compete with the natural protein substrates; 2) the use of farnesyl/geranyl analogs that bind to the enzyme but can't be transferred to the protein substrate (Holstein S, Cermak D, Wiemer D, Lewis K and Hohl R (1998). Phosphonate and bisphosphonate analogues of farnesyl pyrophosphate as potential inhibitors of farnesyl protein transferase. Bioorg Med Chem 6:6 687-94.); and 3) the use of natural products which inhibit enzyme activity (Jayasuriya H, Silverman K, Zink D, Jenkins R, Sanchez M, Pelaez F, Vilella D, Lingham R and Singh S, (1998). Clavaric acid: a triterpenoid inhibitor of farnesyl protein transferase from Clavariadelphus truncatus. J Nat Prod 61:12, 1568-70) through mechanisms that are not clear. Peptide inhibitors are very effective in *in vitro* enzyme assays, but are ineffective in cell assays because they are rapidly degraded by proteases and therefore are of little value as a therapeutic. Rational drug design has been used to generate farnesyl/geranyl analogs to inhibit FPT/GGPT activity. *In vitro,* the analogs appear to inhibit enzyme activity, but are not highly specific for FPT or for certain mutant forms of RAS that need to be selectively targeted. Additionally, the design of the assay makes it impossible to determine whether these analog groups are added to RAS and if modification with analogs facilitates attachment to the membrane. Natural products are randomly screened for inhibitory phenotype. Some natural products have been identified that are inhibitors of FPT or GGPT, but screening is slow and involves numerous sequential assays to determine how the activity and specificity of the target enzyme has been altered. It is not uncommon for drug companies to spend about a decade in exhaustive R&D trying to identify inhibitors of FPT, only to find at the end of the road, that the inhibitor was not specific for the mutated form of RAS.

One would ideally like to rapidly identify drugs that specifically inhibit the farnesylation of constitutively active RAS mutants, but don't affect the modification of wild-type substrates that may be necessary for cell viability. Our strategy solves these problems by enabling mass screening of unlabeled drug candidates in an assay that involves specific forms of RAS.

We have designed an assay that can be readily multiplexed that: 1) incorporates the wild-type target protein and mutants into the initial screening process; 2) directly detects and quantitates the addition of farnesyl/geranyl groups and analogs thereof to the target protein by the enzyme of interest; and 3) can detect differential effects of drug candidates on enzyme activity.

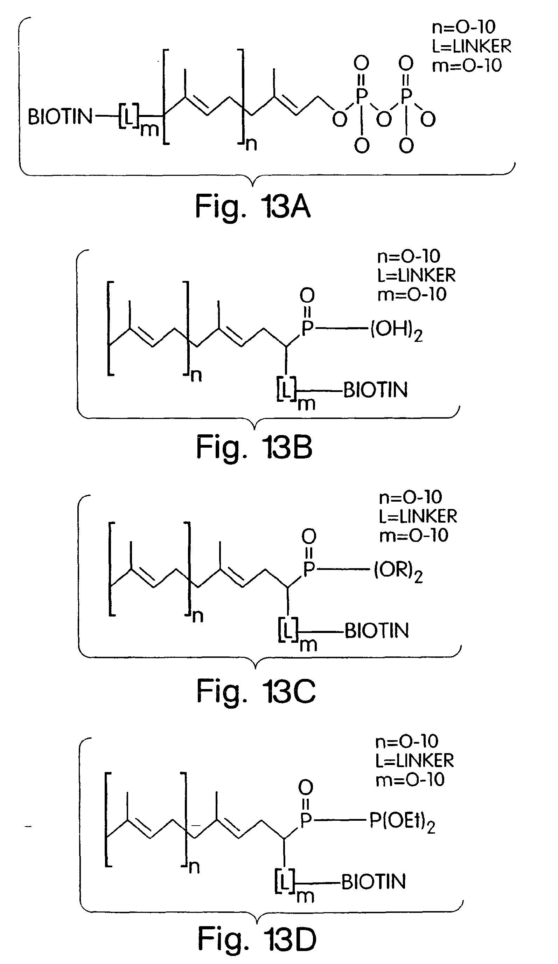

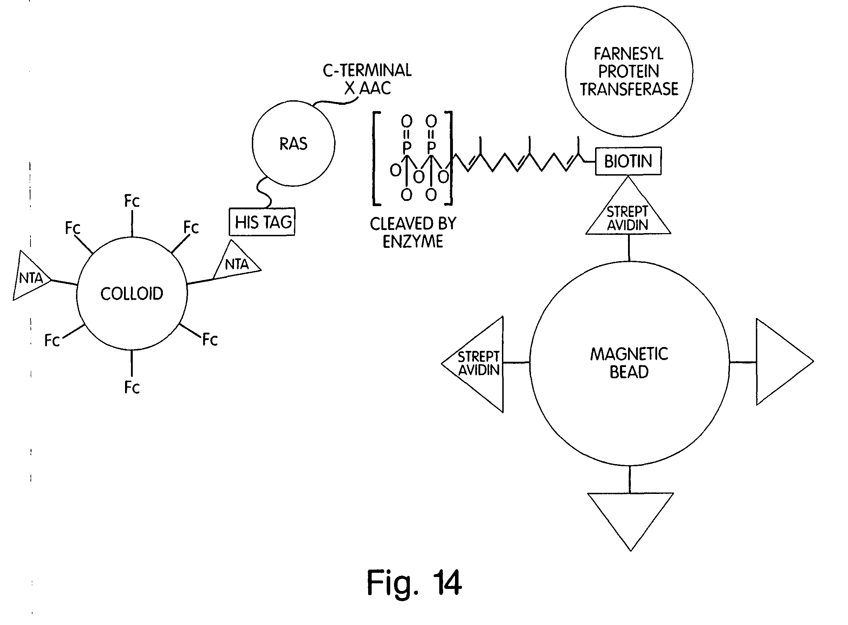

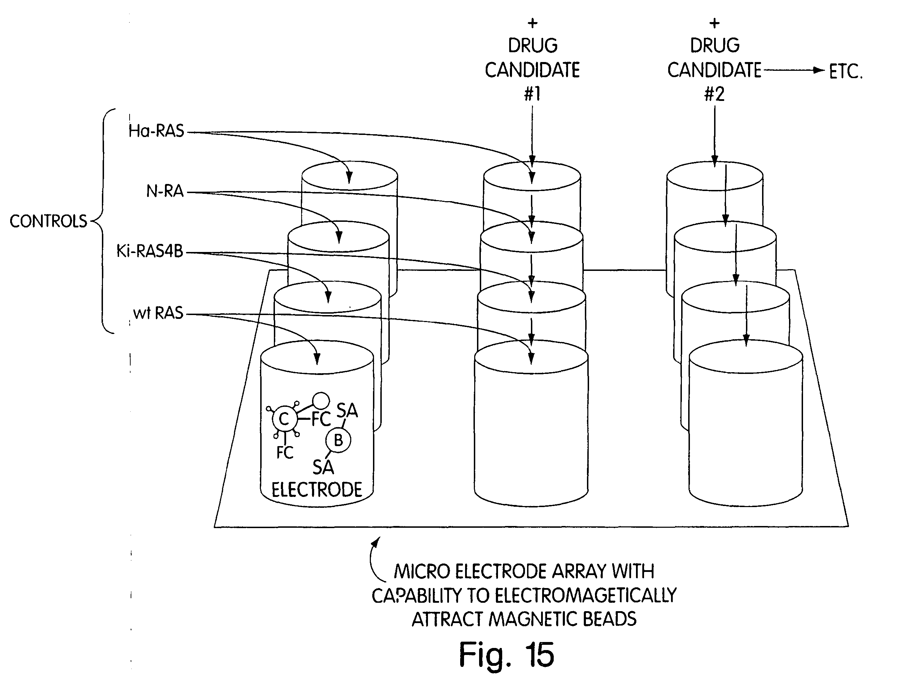

Our strategy is to: 1) attach histidine-tagged RAS proteins (or peptide motifs or fragments derived from the various mutants) to colloids bearing SAMs that present both NTA/Ni(II) groups (to capture histidine-tagged proteins) and transition metals such as ferrocene for electronic signaling; 2) modify farnesyl pyrophosphate derivatives with biotin (Figure 13); 3) add enzyme; 4) add candidate inhibitor; 5) add magnetic beads bearing streptavidin; 6) magnetically attract to a sensing electrode and electronically analyze. The enzyme FPT should add the biotinylated farnesyl (or geranyl) moieties to the RAS immobilized on the signaling colloid. Magnetic beads bearing streptavidin will bind to the biotin and the complex will be both recruitable and detectable. Assays will be performed in parallel in microwells interfaced with a microelectrode array, varying the RAS variant immobilized on the signaling colloid, and the drug candidate (Figures 14 and 15). The entire microelectrode array will be electronically analyzed to determine the differential effects of drug candidates on enzyme activity for particular target proteins. If a drug candidate interferes with the enzyme adding a farnesyl group to the target mutant protein, a diminution of the electronic signal will result.

This strategy can be used to monitor an enzyme's activity or screen for drugs that modulate its activity. Non-histidine-tagged proteins can also be attached to signaling colloids by standard coupling chemistry. Farnesyl derivatives can alternatively be modified with a recognition group other than biotin, that is a binding partner of a group immobilized on the magnetic beads.

Edelstein and Distefano have reported that farnesyl pyrophosphate groups modified with photoactivatable cross-linking moieties can be added to peptide motifs derived from RAS by yeast FPT (Edelstein R. and Distefano M. (1997). Photoaffinity labeling of yeast farnesyl protein transferase and enzymatic synthesis of a RAS protein incorporating a photoactive isoprenoid. Biochem. and Biophys. Res. Comm. 235, 377-382). Their objective was to use this assay to show that farnesyl derivatives were recognized by FPT, by cross-linking the two. This finding is consistent with the idea that farnesyl or garenyl moieties can be modified with biotin at the end distal from the pyrophosphates without interfering with enzyme activity or specificity. derived from RAS by yeast FPT (Edelstein R. and Distefano M. (1997). Photoaffinity labeling of yeast farnesyl protein transferase and enzymatic synthesis of a RAS protein incorporating a photoactive isoprenoid. Biochem. and Biophys. Res. Comm. 235, 377-382). Their objective was to use this assay to show that farnesyl derivatives were recognized by FPT, by cross-linking the two. This finding is consistent with the idea that farnesyl or garenyl moieties can be modified with biotin at the end distal from the pyrophosphates without interfering with enzyme activity or specificity.

### EXAMPLE 2

This example describes an assay to identify unknown targets of known ligands or known therapeutic agents.

The present invention can be used to identify the targets of known biological molecules. For example, certain angiogenesis inhibitors, to treat cancer, have been identified; in most cases, it is not understood how they inhibit angiogenesis. Many of the identified angiogenesis inhibitors are antibodies or proteins that are much more difficult to produce and administer than small molecule drugs. In these cases, considerable effort is directed toward identifying the molecular targets of these therapeutics so that small molecule drug libraries can be directly tested for interaction with the target molecule.

The following is a strategy to identify the physiological target of angiogenesis inhibitor Ai1, which is a protein. Express Ai1 as a histidine-tagged (His-tagged) protein and attach to magnetic beads (or particles) bearing nitrilo tri-acetic acid groups for the capture of the His-tag. The system at this point consists of recruitable particles that bear biospecific ligands for an unknown target molecule(s). The derivatized magnetic beads then are incubated with cell extracts or lysates then magnetically concentrated {magnetic beads can be recovered from a large volume by using a stationary or changing electromagnetic field or by passing the solution over a magnetic column or polymer). At this point, the magnetic beads that present Ai1 should have captured target molecules. Imidazole can then be added to the magnetic beads to chelate the NTA/NiII complex and release the Ai1 and its target. The proteins are then subjected to known analytical techniques such as protein sequencing, gel electrophoresis and the like. Digestive enzymes may be used in the process to release surface bound targets.

Where the therapeutic agent is Ai2, an antibody, one can proceed as above except that Protein A or G (or active fragment of) is attached to the magnetic beads which then capture Ai2. Alternatively, to reduce non-specific interactions, SAMs presenting NTA can be used to capture His-tagged protein A or G.

### EXAMPLE 3

In this example, cells were attached to gold-coated electrodes derivatized with SAMs. The cells, which were still attached to the electrode, were then incubated with a solution containing gold colloids which had been derivatized to present both a ligand specific for a receptor on the cell surface and a redox-active metal capable of delivering an electronic signal to the electrode. After some incubation period, the electrodes were scanned by alternating current voltammetry (ACV). A positive interaction between the colloid-bound ligand and the cell surface receptor will bring the redox-active metal, also on the colloid, close enough to the electrode to transduce an electronic signal.

More specifically, electrodes were derivatized with SAMs to present 10% methyl head groups in a background of 50% Bis(ethynylphenyl thiol) (i.e. C₁₆H₁₀S) to facilitate electron flow to the electrode. 40% triethylene glycol-terminated thiols (HS(CH)₂)₁₁(CH₂CH₂)₃OH) were included to help monolayer packing. It had previously been shown that cell growth can be supported on HSC15-methyl-terminated SAMs that were coated with collagen. Both the HSCH₂C₁₅CH₃ and the collagen are insulating molecules and can inhibit electron flow to the electrode. For this reason, in this example, the saturated carbon chain-collagen coverage was reduced to produce islands of growing cells adjacent to the more conducting molecular wires. Human endothelial cells (HUVECs), that present a cell surface receptor αVβ3, which is important for angiogenesis, were grown on the electrodes. SAM-coated gold colloids bearing a ligand for the receptors and ferrocene moieties for electronic signaling were briefly incubated with the cell-presenting electrodes, then analyzed by ACV.

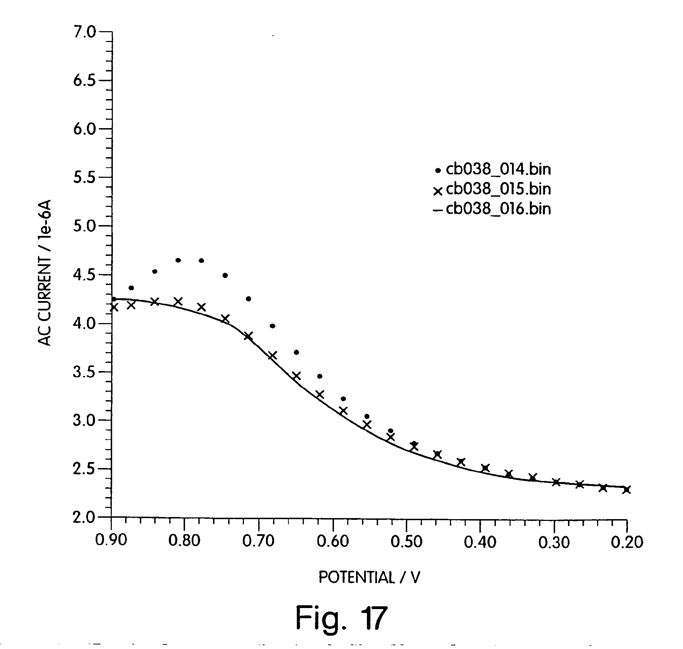

For ACV analysis, a 1ml capacity silicone gasket was clamped onto the cell-presenting electrode. 100ul of NTA-Ni colloids that had been pre-bound with a His-tagged RGD motif peptide and 100ul PBS were added to the gasket for incubation with the cell-presenting electrode. After 15 minutes, the first ACV scan was taken. Two successive scans were taken at 15 minute intervals. Current output was plotted against were identical (Figure 17). In sharp contrast to the 1.6 uAmp peak for the positive, no well-defined peak resulted for the negative control.

### Example 4: Cell growth on conducting surfaces

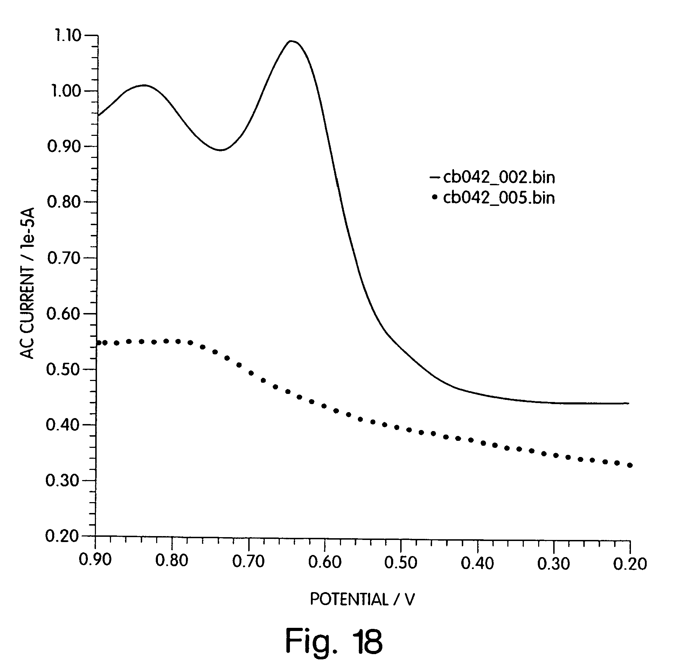

This example describes the electronic detection of cells grown on "conducting" surfaces that were not coated with collagen. Cells were grown on gold electrodes that were modified with sulfur-containing molecules, in some cases assembled into monolayers, but not coated with collagen. Electrode modification was performed as described in the electrode preparation section of Example 3, with the exception that electrodes incubated with 100% candidate molecule were not heat cycled in tri-ethylene glycol-terminated thiol. Several electrodes were assembled in the same cell growth flask and media containing HUVEC cells was added. The electrodes and cells were incubated in a CO₂ incubator for 24 hours. Surfaces were visually analyzed using 100X magnification. Table 1 lists surface modification and subsequent cell adhesion/growth characteristics. Surfaces showed low non-specific binding. Once cells bound, however, cell growth was good. Cells can easily be immobilized on a SAM by presenting a metal chelate coordinating a metal via the SAM, linking a protein to the SAM via a metal binding tag on the protein, the protein attracting the cell. Photographs were taken to document results (not shown). Cells were incubated with colloids (as described above) that displayed ferrocene moieties and a peptide, HHHHHH(S₄G₁)₃GRGDSGRGDS, that was specific for the alpha V beta 3 receptor on the cell surface; or as a negative control, an irrelevant peptide, HHHHHH-Glutathione S-Transferase (GST). Figure 18 shows that cells grown on a 100% ethynylphenyl thiol (MF1) SAM-coated electrode produced current peaks only if incubated with colloids bearing the ligand specific for the alpha V beta 3 receptor (Figure 18, solid line) and not when incubated with colloids derivatized with an irrelevant peptide GST (Figure 18, dotted line).

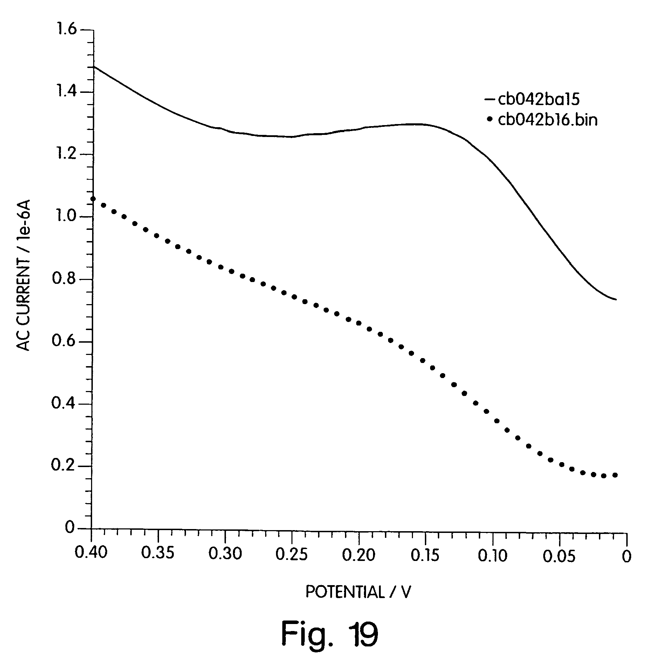

Figure 19 is an electrochemical scans of electrodes derivatized with 25% 2-mercaptoethylether in a background of insulating tri-ethylene glycol terminated thiols. Cells derivatized with colloids presenting the RGD sequence peptide produced a small peak (Figure 19, solid line), while the cells incubated with colloids presenting the GST peptide did not (Figure 19, dotted line).

**TABLE I**

| **SURFACE MODIFICATION** | **CELL ADHESION within 1/2 hour** | **CELL GROWTH within 24 hours** |
|---|---|---|
| 100% methyl-terminated SAM | NO | NO |
| 100% methyl-terminated SAM, incubated with irrelevant proteins | NO | NO |
| 100% methyl-terminated SAM, incubated with collagen | NO | YES |
| 10% methyl terminated thiol in a background of molecular wire thiols, with or without tri-ethylene glycol-terminated thiols then coated with collagen | NO | YES |
| 100% tri-ethylene glycol-terminated SAM | NO | YES |
| 100% HS-2-unit molecular wire | NO | YES |
| 100% 2-mercaptobenzothiazole | NO | YES |
| 100% 1,2-benzenedimethanethiol | NO | YES |
| 100% Benzene ethane thiol | NO | YES |
| 100% 2-mercaptoethyl ether | NO | YES |
| NTA-Ni(II) in a background of tri-ethylene glycol-terminated thiol with or without ferrocene thiols mixed in- unbound by a His-tagged peptide | NO | YES |
| NTA-Ni(II) in a background of tri-ethylene glycol-terminated thiol with or without ferrocene thiols mixed in - bound by an irrelevant His-tagged peptide | NO | NO |
| NTA-Ni(II) in a background of tri-ethylene glycol-terminated thiol, with or without ferrocene thiols mixed in - bound by a cell specific His-tagged peptide | YES | YES |

### Example 5: Decoration of polymeric bead with colloid particles via Glutathione-S-Transferase / Glutathione binding

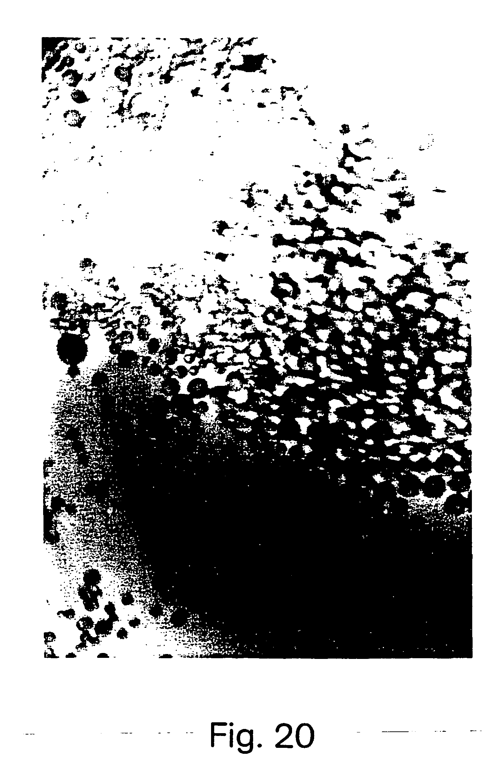

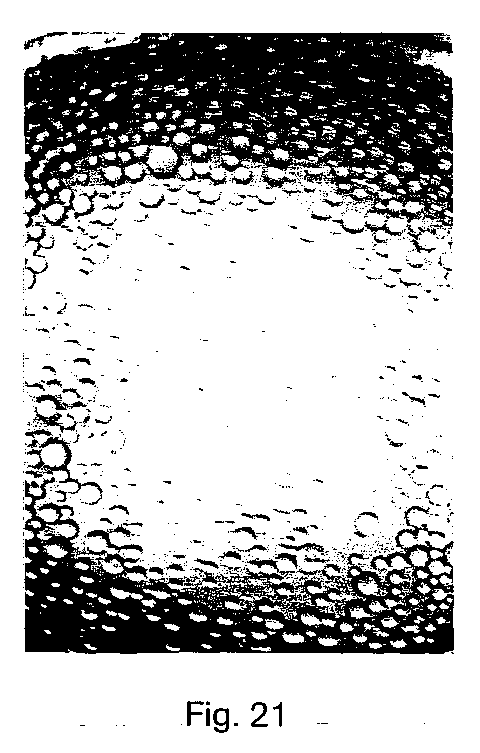

See Figures 20 and 21. The example below demonstrates how colloids presenting a target protein agglomerate onto a non-colloidal particle that presents a small molecule, or drug candidate, that is a binding partner of the target protein. Many combinatorial drug libraries are synthesized on non-colloidal particles or beads and can be mixed with colloidal particles that display a medically relevant target species. One can readily identify the bead that displays the drug that is a binding partner for the target species, as the colloidal particles agglomerate onto that bead and color it red.

A target protein, Glutathione-S-Transferase (GST) was histidine-tagged and immobilized on SAM-coated colloids that presented NTA-Ni (histamine tags bind NTA-Ni). 30ul of colloids presenting 40uM NTA-Ni on the surface were added to 65ul of 21.5uM GST, to give a final concentration of 14uM GST in solution. Glutathione, a small molecule that binds GST, is commercially available bound to agarose beads through Sigma-Aldrich. Glutathione-coated
beads were incubated with the solution of GST-bound colloids. Within minutes, the GST bound to the glutathione beads, bringing the colored colloids out of solution, and decorating the beads red (Figure 20). Beads displaying a small molecule that does not bind to GST remained colorless
when incubated with the GST-bound colloids (Figure 21). A second negative control, in which glutathione-coated beads were incubated with 30ul NTA-Ni colloids in the absence of GST showed that NTA-Ni-colloids do not bind nonspecifically to the bead surfaces or to the glutathione.

### Example 6: Demonstration of Control of SAM permeability to electrons

This example demonstrates the ability to form a SAM including enhanced electronic communication. The SAM is formed on a surface that includes a mixture of a first, tight-packing species and a second species of different molecular structure that enhances the permeability of the SAM to electronic communication. A defect, or opening, is formed in the SAM allowing fluid to which the surface is exposed to communicate electrically with the surface. Specifically, certain small sulfur containing compounds having disruptive structures relative to the SAM as a whole were stably incorporated into a SAM. and greater permeability to electrons was demonstrated. This example demonstrates that a surface can be made electrically relatively conductive, and then support cell growth.

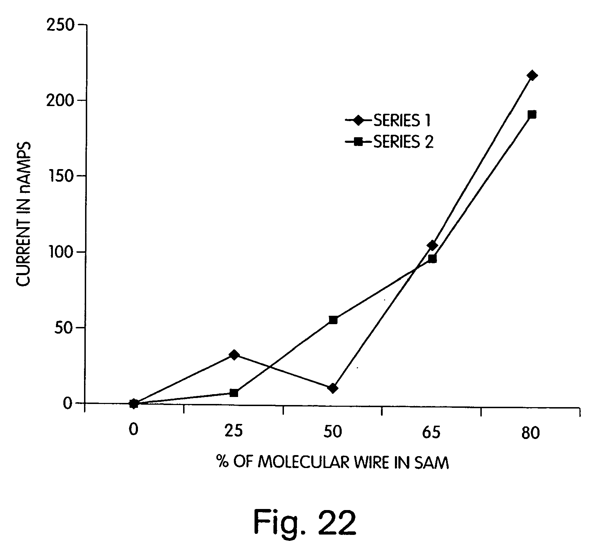

A water-soluble ferrocene derivative was dissolved in the electrolyte solution: 100mM solution of ferrocenedicarboxylic acid in 500uM NaClO4. The working electrode was a gold-coated electrode derivatized with a SAM comprised of varying amounts of 2-unit molecular wire (MFI). The height of the peak at a characteristic ferrocene potential was plotted as a function of molecular wire density. As a negative control, a gold-coated electrode was derivatized with an insulating SAM comprised of 100% tri-ethylene glycol terminated thiol. Figure 22 shows that the "conductivity" of the SAM or the ability of electrons in solution to penetrate the SAM is a function of the density of 2-unit molecular wire integrated into the SAM. This system was used to test the conductivity of electrodes modified with a panel of sulfur-containing compounds. The compounds were dissolved in DMF at 50% candidate compound and 50% tri-ethylene glycol terminated thiol. Electrodes were derivatized as described in Example 1. Table 2 lists the candidate compounds and the height of the current peak produced when analyzed by ACV. Figure 22 shows two experimental results of tests of conductivity of a monolayer as a function of monolayer disruption by poly (ethynylphenyl thiol).

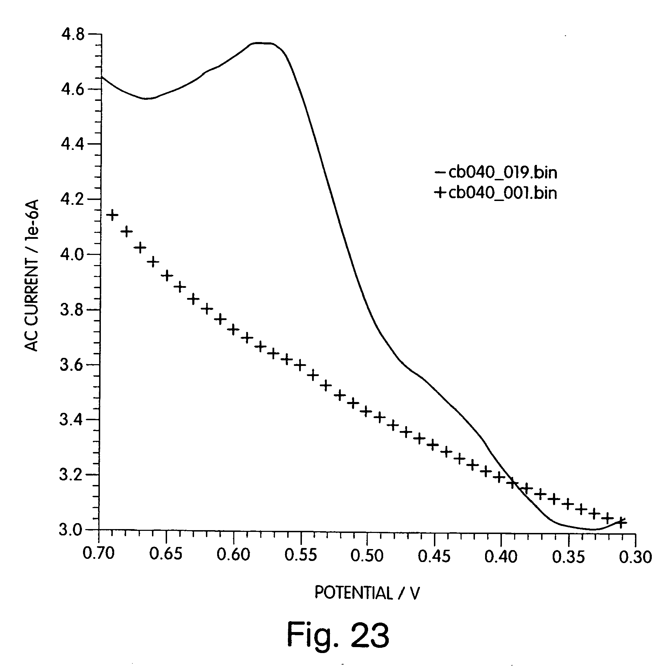

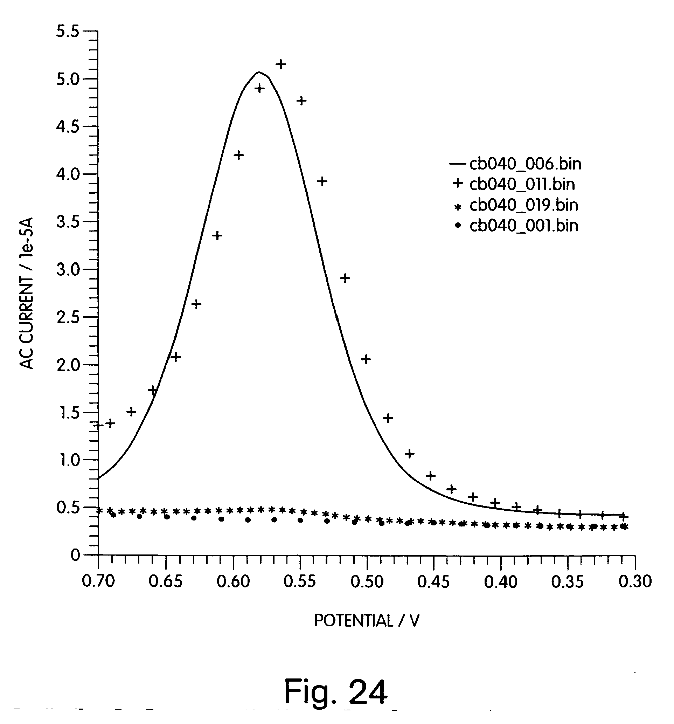

SAMs were formed on gold chips from 500 micro molar triethylene glycol-terminated thiol and 500 micro molar of either mercaptobenzothiazole or 2-mercaptoethyl ether in DMF. The chips were clamped between a flat substrate and a 1 ml capacity silicon gasket. A solution of ferrocene dicarboxyllic acid was dissolved in 500 micro molar NaClO4 and placed in the silicon gasket with a Ag/AgCl reference electrode and a Pt auxiliary electrode. The gold chip was connected as the working electrode. The system was analyzed by ACV. The magnitude of the current peaks, resulting from the ferrocene in solution communicating with the electrode, was an indicator of the ability of the trial compounds to make the SAM more permeable to electron flow by creating defects within the SAM. Figure 23 is an overlay of a SAM comprised solely of the insulating species tri-ethylene glycol-terminated (CH2)11-SH (+++ line) and the reportedly more conducting poly-ethynylphenyl thiol species (MF1) (solid line). The conducting poly-ethynylphenyl thiol species is expected to by more conductive, as an identical molecule but with two additional repeat units is reported to be conductive (Science 1997 Bumm et al). As predicted, the alkyl thiol SAM does not produce a current peak in a ferrocene dicaroxyllic acid solution, but a SAM comprised of 50% poly-ethynylphenyl thiol does. Figure 24 shows the plots of Figure 23 (dots are TEG-terminated thiol, stars are poly-ethynylphenyl thiol), against the more conducting SAMs including 2-mercaptoethyl ether (solid line) and mercaptobenzothiazole (+++ line), showing that the current peak from the poly-ethynylphenyl SAM is orders of magnitude smaller than those generated by SAMs comprised of 50% 2-mercaptoethyl ether or a SAM comprised of 50% mercaptobenzothiazole, which is consistent with the idea that SAMs can be made permeable to electron flow by either incorporating conductive species into the SAM or by inserting "defect" or "opener" molecules into the SAM.

**TABLE 2. Electrodes were derivatized with the following compounds in a background of tri-ethylene glycol-terminated thiol. Electrode preparation was performed as described in Example 1. The "conductivity" or permeability of the surfaces was assayed by measuring the magnitude of the current peak produced by the oxidation/reduction of ferrocene dicarboxyllic acid in the electrolyte solution (0.5 M NaClO4). The ability of each compound to resist non-specific binding was assayed by dipping each surface in BSA (bovine serum albumin) prior to measurement. Non-specifically adsorbed proteins would occlude the conduction of electrons through the monolayer to the electrode.**

| **COMPOUND** | **50% coverage Peak height in :Amps** | **25% coverage Peak height in :Amps** | **25% coverage Peak height in :Amps after blocking with BSA** | **Supports cell growth on 100% coverage or fractions thereof** |
|---|---|---|---|---|
| HS(CH₂)₁₁Tri-ethylene glycol **100% coverage-negative control** | 0 | ND | ND | YES |
| HS-2-unit molecular wire **Positive control** | 0.415 | ND | 0.15 at 50% coverage | YES |
| 2-mercaptopyridine | 14.70 | ND | ND | ND |
| 2-mercaptobenzothia zole | 44.00 | 0.28 | 0.29 | YES |
| Dithiothreitol | 41.00 | ND | ND | ND |
| 1,2-benzenedithiol | 40.00 | ND | ND | ND |
| 1,2-benzenedimethanet hiol | 34.90 | 28.00 | 4.02 | YES |
| Benzeneethanethio 1 | 31.50 | 0.51 | 0.08 | YES |
| 2-mercaptoethyl ether | 39.30 | 57.70 | 19.0 | YES |

### Example 7: Detection of Protein-protein interactions

This example demonstrates the utility of a colloid particle having an immobilized signaling entity and an immobilized protein. (See Figure 27.)

Histidine-tagged Glutathione-S-Transferase (GST-His) was attached to NTA-SAM-coated colloids, displaying 40uM NTA-Ni and 100uM ferrocene-thiol. Commercially available magnetic beads presenting protein A were coated at 1/10 binding capacity with anti-GST antibody, added at a 1:5 ratio to the GST-colloids, and measured on a 50% MF-1 SAM-coated electrode, which was placed on top of a magnet. The magnet pulled the magnetic beads onto the electrode surface to form a thick, visible precipitate. The GST-colloids were brought down to the electrode surface by the interaction with the GST-antibody on the magnetic beads to give a current peak at approximately 280mV. Two negative controls were run, one where GST was not attached to the colloid surface, and another where the GST antibody was not attached to the magnetic beads. Neither negative control gave a current peak. Figure 25 plots the results of this demonstration. Solid line represents interaction between GST-His-presenting colloids and anti-GST/Ab on magnetic beads. Open circles represent magnetic beads presenting the antibody incubated with colloids that did not present GST. Closed circles represent beads not presenting the antibody, incubated with colloids that presented GST.

### Example 8: Cell Detection

This example demonstrates both the advantage of forming a SAM on a surface that includes a mixture including a molecular species that enhances electronic communication across the SAM by forming a defect in the SAM allowing fluid to which the surface is exposed to communicate electrically with the surface, and the utility of attachment of a colloid carrying immobilized signaling entity to a protein. The protein is in turn immobilized at a cell attached to the surface of an electrode presenting the SAM. The defect in this case is caused by bulk of the a SAM-incorporated molecule including phenyl rings.

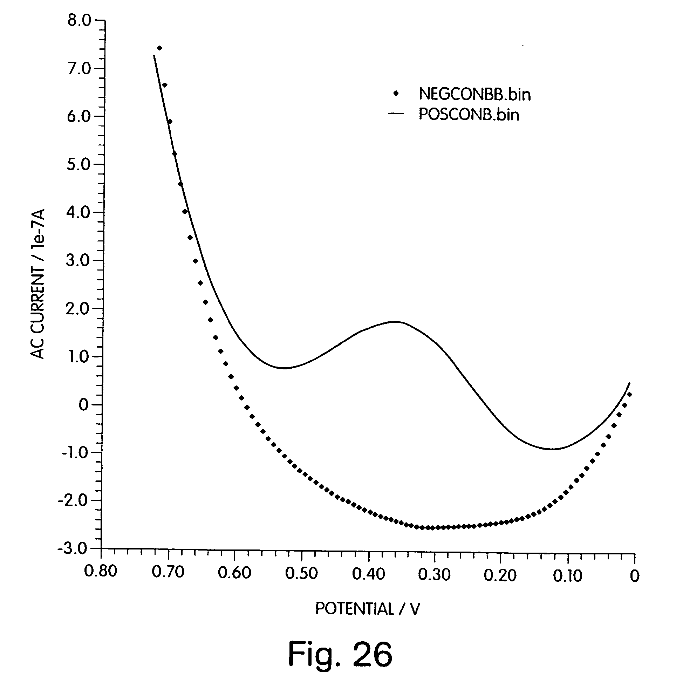

HUVEC cells were suspended in media and placed in a flask over a SAM coated on a gold surface. The SAM included 50% straight chain thiols, and 50% of the 2-unit poly (ethynylphenyl) thiol (MF1). 5ul of an 8.4mM RGD-His peptide solution was added to the media, and cells were incubated at 37C overnight to adhere to the electrode surfaces. After approximately 16 hours, 100ul of SAM-coated colloids, displaying NTA for capturing the RGD-His peptide and ferrocene for signaling, were added to the cells and incubated for 20 min at room temperature. The electrodes were then rinsed in buffer to wash off any unbound colloids and measured. Current peaks were recorded at 220-250mV. Negative controls were cells incubated with His-GST, an irrelevant protein that should not bind to cells. Colloids were added to negative controls, electrodes were rinsed in buffer, and measurements were taken. No peaks were observed for negative controls. Figure 26 shows a peak (solid line) generated when colloid presenting ferrocene signaling entity and his-tagged ligand to a cell surface receptor is brought to an electrode surface by cell/surface interaction. Diamond represent negative control where colloids displayed an irrelevant protein selected not to bind to the cell surface receptor.

### Example 9: Detection of Protein/Ligand Interaction

This example demonstrates the ability to determine protein/ligand interaction in the absence of SPR without labeling either the protein or the ligand. Specifically, exposing a ligand to a protein suspected of interacting with the ligand where the ligand is in fixed proximal relationship with an electroactive entity, namely ferrocene, whose electroactive signal is dependant upon proximity to the protein, by forming a SAM including the ligand and ferrocene on the surface of an electrode.

### Example 10: Detecting Ligand-Receptor Interactions for Unmodified Ligands

We have found that the characteristic oxidation potential of a ferrocene derivative can be shifted based on the chemical nature of the ferrocene derivative's local environment. As proteins are brought into close proximity to the ferrocene derivative, a second current peak at an altered potential appears. The magnitude of this altered peak is proportional to the density of protein in the system.

A panel of gold-coated electrodes were derivatized with heterologous SAMs that comprised a constant density of conductive molecular wire thiol and variable density of methyl-terminated thiol in a background of tri-ethylene glycol-terminated thiol. It had previously been shown that: 1) tri-ethylene glycol-terminated SAMs resist the non-specific adsorption of proteins; and 2) methyl-terminated SAMs bind proteins, specifically collagen. We therefore assumed that if the electrodes are incubated with collagen in solution, the density of collagen, that non-specifically adsorbs, will increase as the density of methyl-terminated thiol increased. SAMs that contained 2%, 4%, 10% and 15% methyl-terminated thiol were formed on gold-coated electrodes. Half the electrodes were incubated for 2 hours at 4°C with 0.005 mg/ml collagen in PBS while the other half was incubated with PBS alone. The electrodes were analyzed by alternating current voltammetry (ACV) with a water soluble ferrocene derivative, ferrocenedicarboxyllic acid, dissolved in the electrolyte solution (0.5 M NaClO4). The alternating oxidation/reduction of the ferrocene at a characteristic potential produces a current peak. The magnitude of this current peak and potential at which it occurred were recorded. At low density methyl-terminated thiol, a current peak was produced at about 450 mVolts; its magnitude and position appeared to be unaffected by the presence of the protein, collagen. At higher density methyl-terminated thiol, 10% and over, a second current peak is produced at about 300 mVolts, but only in the presence of the protein collagen. Figure 28 shows high-density methyl terminated thiol electrodes in the presence of ferrocene dicarboxylic acid. Solid line represents electrodes with collagen bound to the methyl thiols. The hydrophobic environment caused by the collagen on the surface of the electrode causes the oxidation potential of the ferrocene to shift, and two peaks are seen. Solid circles represents high-density methyl terminated thiol electrodes that are not bound to collagen and thus do not affect the oxidation of the ferrocene.

This strategy can be used to detect the presence of unmodified proteins, peptides, cells as follows: a mixed SAM is formed from ferrocene dicarboxyllic acid attached to an alkyl thiol, a thiol designed to make the SAM permeable to electron flow and a thiol terminated in a binding partner for a target species (the binding partner can also be histidine-tagged and attached to the electrode by binding to an NTA-Ni moiety in the SAM). If the target species is present in the sample solution, then it binds to its binding partner that is presented on the SAM. The presence of the protein near the SAM surface changes the chemical environment around the ferrocene dicarboxyllic acid and causes its oxidation potential to shift to a lower potential. A shift in the characteristic oxidation potential of the immobilized redox-active metal indicates the presence of the target species.

When these experiments were performed with ferrocene derivatives that did not have polar substituents on the ring, no effect was observed. Therefore, for this strategy to work, it is essential that the immobilized redox-active metal is in a polar environment and that the immobilized binding partner (connected to the SAM via a thiol) is as small as possible.

### Example 11: ELISA

A technique familiar to those skilled in molecular or cellular biology is Enzyme-linked Immunosorbent Assays (ELISA) (Current Protocols in Molecular Biology, Volume 2, Immunology 11.2, 1996, copyright from John Wiley and Sons Inc. 1994-1998). A major problem with ELISAs is sensitivity. Enzyme signaling occurs at a 1:1 ratio; one antibody-coupled enzyme signals the presence of one antigen. Therefore, microgram quantities of antigen are necessary for an effective ELISA, which is not plausible in all cases. This renders ELISAs ineffective for detection of antigens on a cell surface that are expressed or presented at low levels. Another problem with ELISAs is that at low antigen concentrations, the assay is very time-consuming. The amount of enzyme hydrolysis is directly proportional to the time of hydrolysis. Normally when one performs an ELISA, a target species is directly or indirectly attached to a plastic substrate. The presence of the immobilized species is then detected by binding to it a "secondary" antibody that also has a signaling capability; the secondary antibody is usually conjugated to an enzyme, typically horseradish peroxidase (HRPO), alkaline phosphatase (AP), or a fluorescent tag which is capable of performing a reaction on an added substrate that results in a color change (detected by a spectrophotometer), or a fluorescence labeling tag that can be detected by a fluorimeter (see, for e.g., "Localization of a passively transferred human recombinant monoclonal antibody to herpes simplex virus glycoprotein D to infected nerve fibers and sensory neurons in vivo", Sanna PP, Deerinck TJ, and Ellisman MH ,1999, Journal of Virology Oct. Vol 73 (10 8817-23)). Most often, for convenience, so that every antibody does not need to be conjugated to an enzyme, a mouse antibody is used as the specific recognition antibody, then an enzyme-conjugated rabbit-anti-mouse antibody is added.

Using the technology described herein, one can greatly increase the sensitivity of ELISAs and detect the presence of the immobilized target species using a natural ligand (protein or peptide) or a drug candidate as the probe molecule. The presence of the immobilized species is detected by binding to it a *ligand* attached to a colloid that also presents a plurality of horseradish peroxidase (HRPO) or alkaline phosphatase (AP). The enzyme can be conveniently linked to the colloid by a variety of means, including a histidine-tag attached directly to the enzyme, or by binding a mouse-anti-goat enzyme- conjugated antibody to a goat antibody that is attached to the colloid via a histidine-tagged protein G (Akerstom, B., Nielson, E., Bjorck, L. Jounal of Biological Chemistry, 1987 Oct. 5 Vol. 262 (28); pgs. 13388-91 and Fahnestock, S.R., Alexander, P., Nagle, J. and Filpula, D. (1986) Journal of Bacteriology Vol. 167, 870-880). By binding a ligand co-immobilized on a colloid with a plurality of enzymes, to the target species *in place of* a secondary antibody, the ratio of signaling molecules to binding events is increased by orders of magnitude. The binding of one antibody or ligand on the colloid to a presented antigen on an ELISA plate indirectly results in the binding of thousands or millions of enzymes. Alternatively, a known species can purposely be attached to wells of a 96-well plate so that one can probe with colloids that each present a separate drug candidate along with the signaling enzymes. Currently, it is not possible to do this with existing ELISA technology each drug candidate can not be conjugated to an enzyme. Alternatively, the natural ligand for the immobilized target can be presented on the colloid along with the signaling enzymes and drug candidates added to each well of the plate to disrupt the interaction. Unbound colloids and thus their signal are lost in a wash step. The target for the antibody- or ligand-presenting colloids can be a cell or a protein bound directly or indirectly (via another antibody or ligand) to an ELISA plate. The advantage to this modification of an ELISA is not only sensitivity, but also efficiency. Because several hundred signaling enzymes remain bound via the colloids to one antigen, substrate hydrolysis will occur more quickly, and less time will be needed for an adequate reading.

## Claims

1. A method comprising:
exposing a biological or chemical agent, which is linked to a non-colloidal structure, to a binding partner of the biological or chemical agent, which is non-covalently linked to a colloid particle via a metal binding tag/metal/chelate linkage;
allowing the colloid particle to fasten to the non-colloidal structure *via* specific interaction between the biological or chemical agent and the binding partner; and
determining fastening of the colloid particle to the non-colloidal structure;
wherein the colloid particle is derivatized with a self-assembled monolayer.

2. A method as claimed in claim 1, wherein the colloid particle is a gold colloid particle.

3. A method as claimed in claim 1 or 2, wherein the non-colloidal structure is an electrode or bead.

4. A method as claimed in claim 3, wherein the non-colloidal structure is a bead, preferably wherein the bead comprises polymeric material, agarose, tentagel, and/or magnetic material, preferably wherein the bead is a polystyrene bead.

5. A method as claimed in any of the preceding claims, wherein the colloid particle comprises an auxiliary signalling entity.

6. A method as claimed in claim 5, wherein the auxiliary signalling entity comprises a dye, pigment, electroactive molecule, fluorescent moiety, up-regulating phosphor, or enzyme-linked signalling moiety including horse radish peroxidase and alkaline phosphatase.

7. A method as claimed in claim 5 or 6, wherein the signalling entity comprises a metallocene, preferably ferrocene or a ferrocene derivative.

8. A method as claimed in claim 7, wherein the non-colloidal surface is a magnetic bead, and determining fastening comprises magnetically drawing the bead into proximity with an electrode, and determining proximity of the metallocene to the electrode by activating the electrode.

9. A method as claimed in any of the preceding claims, comprising allowing a plurality of colloid particles to fasten to the non-colloidal structure, and determining fastening of the plurality of particles to the non-colloidal structure, preferably wherein the plurality of colloid particles comprises auxiliary signalling entities, preferably wherein the auxiliary signalling entities comprise dyes, pigments or electroactive molecules.

10. A method as claimed in any of the preceding claims, wherein the agent and the binding partner biologically bind to each other.

11. A method as claimed in any of the preceding claims, wherein at least one of the agent and the binding partner is an antibody.

12. A method as claimed in any of the preceding claims, wherein the biological or chemical agent is:
(a) a drug candidate, and the binding partner is a target of the drug candidate;
(b) a nucleic acid sequence;
(c) a peptide; or
(d) a protein.

13. A method as claimed in any of the preceding claims, comprising determining, preferably by visual inspection, immobilization of the particle on the non-colloidal structure by determining a change in spectrum of absorbed or transmitted electromagnetic radiation interacting with the particle.

14. A method as claimed in any of the preceding claims, comprising allowing the colloid particle the ability to fasten to the non-colloidal structure in the presence of a candidate drug for interruption of the interaction between the agent and the binding partner.

15. A method as claimed in any of the preceding claims, wherein the non-colloidal structure is a bead, further comprising providing a plurality of beads, a plurality of biological or chemical agents adapted for linkage to the beads, a plurality of colloid particles, and a plurality of binding partners of the biological or chemical agents adapted for linkage to the particles, wherein at least some of the agents and the binding partners are suspected of having the ability to bind to each other, the method comprising exposing at least some of the beads to at least some of the particles, and determining immobilization of the particles on the beads.

16. A method as claimed in claim 15, wherein the biological or chemical agents are drug candidates and the binding partners are targets of the drug candidates, the method comprising:
(a) providing at least a first and a second bead in two separate locations, each carrying a different immobilized drug candidate, exposing the plurality of colloid particles to the at least two beads, and differentiating linkage of the colloid particles to the first bead versus the second bead, preferably wherein the at least two beads are separately located in at least two different wells of a multi-well plate; or
(b) providing at least two beads carrying a drug candidate in two separate locations, exposing the first bead to a first set of colloid particles carrying a first target of the drug candidate, and exposing the second bead to a second set of colloid particles carrying a second target of the drug candidate, and differentiating linkage of the first set of colloid particles to the first bead versus the second set of colloid particles to the second bead.

17. A method as claimed in any of the preceding claims, comprising:
providing at least a first and a second non-colloidal structure comprising polymeric beads and at least a first and a second agent linked to the first and second beads, respectively;
providing a plurality of colloid particles each carrying immobilized thereto a suspected binding partner of the first and/or second agent;
exposing the beads to the particles; and
differentiating linkage of the particles to the first bead versus the second bead;
preferably wherein the first and second agents linked to the first and second polymeric beads are suspected of biological or chemical interaction with the binding partner, and the differentiating step comprises differentiating interaction between the first agent and the binding partner versus the second agent and the binding partner.

18. A method as claimed in any of the preceding claims, comprising:
providing a plurality of non-colloidal structures comprising beads each carrying the agent immobilized thereto;
providing a first set and a second set of colloid particles, the first set each carrying immobilized thereto a first suspected binding partner of the agent and the second set each carrying immobilized thereto a second suspected binding partner of the agent;
exposing a first bead to the first set of colloid particles and a second bead to the second set of colloid particles; and
differentiating linkage of the first set of colloid particles to the first bead versus the second set of colloid particles to the bead;
preferably wherein the first and second suspected binding partners are suspected of biological or chemical interaction with the agent, and the differentiating step comprises differentiating interaction between the agent and the first suspected binding partner versus the agent and the second suspected binding partner.

19. A method as claimed in any of claims 1, 2 or 5 to 14, wherein the non-colloidal structure is a cell.

20. A method as claimed in claim 19, wherein the agent is a receptor at a surface of the cell and the binding partner is a ligand for the receptor.

21. A method as in claim 20, wherein the ligand is a peptide, protein, antibody, enzyme, or chemical species.

22. A method as claimed in any of claims 19 to 21, comprising determining immobilization of the colloid particle to the cell:
(a) by determining a change in spectrum of absorbed or transmitted electromagnetic radiation interacting with the particle, preferably by visual inspection; or
(b) electronically, preferably *via* alternating current voltammetry.

23. A method as claimed in claim 19, wherein the non-colloidal structure is a cell exposing a receptor at a surface thereof, further comprising providing a ligand for the receptor adapted for linkage to the colloid particle and exposing the ligand and the colloid particle to the cell in the presence of a candidate drug for disruption of interaction between the ligand and the receptor; and determining fastening of the colloid particle to the cell, preferably, wherein the determining step involves determining inhibition of fastening of the colloid particle to the cell indicative of effectiveness of the candidate drug in disrupting receptor/target protein interaction, preferably, comprising providing at least a first and a second cell in separate locations, exposing the first cell to the ligand for a cell receptor of the first cell and a colloid particle, the ligand adapted for linkage to the colloid particle, and a first candidate drug for disruption of interaction between the ligand and the receptor, and adding to the second cell the ligand and the colloid and a second candidate drug for disruption of interaction between the ligand and the receptor, and differentiating linkage of the colloid particle to the first cell versus the second cell.

24. A method as claimed in claim 19, wherein the non-colloidal structure is a cell exposing a receptor at a surface thereof, further comprising:
providing at least two of the cells in separate locations;
exposing each of the cells to a different target protein to a cell receptor and a colloid particle adapted for linkage to the respective target protein; and
determining fastening of the colloid particles to the cells indicative of binding of the different target proteins to the cell receptors.

25. A method as claimed in any of the preceding claims, wherein an enzyme having the ability to cleave the agent or binding partner is present.

26. A method as in claim 25, wherein a candidate drug for moderation of activity of the enzyme is present, preferably, wherein the binding partner comprises a protein or peptide that can be cleaved by the enzyme, preferably, wherein the protein is adapted for linkage to the colloid particle and to the non-colloidal surface

27. A method as in claim 1, wherein the colloid particle and the non-colloidal structure are exposed to a substrate for an enzyme adapted for linkage to the non-colloidal structure, a molecular species linkable to the substrate *via* enzyme activity adapted for linkage to the colloid particle, and an enzyme for the substrate, preferably further comprising exposing the colloid particle and the non-colloidal structure to a candidate drug for moderation of activity of the enzyme.

28. A method as in claim 27, wherein the non-colloidal structure is a magnetic bead and the signalling entity is a metallocene, further comprising magnetically drawing the bead into proximity with an electrode, and determining proximity of the metallocene to the electrode by activating the electrode thereby determining effectiveness of the drug candidate in moderation of activity of the enzyme.

29. A method as in claim 27, wherein the non-colloidal structure is a surface of an electrode and the colloid particle carries an immobilized electroactive entity, and the method comprises determining proximity of the electroactive entity to the electrode by activating the electrode thereby determining effectiveness of the drug candidate in moderating activity of the enzyme.

30. A composition comprising:
a biological or chemical agent, linked to a non-colloidal structure; and
a binding partner of the biological or chemical agent, non-covalently linked to a colloid particle via a metal binding tag/metal/chelate linkage;
wherein the colloid particle is capable of fastening to the non-colloidal structure *via* specific interaction between the biological or chemical agent and the binding partner;
wherein the colloid particle is derivatized with a self-assembled monolayer.

31. A composition as claimed in claim 30, wherein a signalling entity is immobilized to the colloid particle, preferably wherein the signalling entity is an electroactive entity.

## Patentansprüche

1. Verfahren, umfassend:
In-Kontakt-Bringen eines biologischen oder chemischen Stoffes, der mit einer nicht-kolloidalen Struktur verbunden ist, mit einem Bindungspartner des biologischen oder chemischen Stoffes, der über eine Verbindung aus metallbindender Markierung/Metall/Chelat nichtkovalent mit einem Kolloidpartikel verbunden ist,
Zulassen, dass sich das Kolloidpartikel über spezifische Wechselwirkung zwischen dem biologischen oder chemischen Stoff und dem Bindungspartner an die nicht-kolloidale Struktur heftet, und
Bestimmen des Anheftens des Kolloidpartikels an die nicht-kolloidale Struktur,
wobei das Kolloidpartikel mit einer selbstorganisierenden Monoschicht derivatisiert wird.

2. Verfahren nach Anspruch 1, wobei das Kolloidpartikel ein Gold-Kolloidpartikel ist.

3. Verfahren nach Anspruch 1 oder 2, wobei die nicht-kolloidale Struktur eine Elektrode oder ein Kügelchen ist.

4. Verfahren nach Anspruch 3, wobei die nicht-kolloidale Struktur ein Kügelchen ist, wobei das Kügelchen vorzugsweise Polymermaterial, Agarose, Tentagel und/oder magnetisches Material ist, wobei das Kügelchen vorzugsweise ein Polystyrol-Kügelchen ist.

5. Verfahren nach einem der vorhergehenden Ansprüche, wobei das Kolloidpartikel eine Zusatzsignalisierungseinheit umfasst.

6. Verfahren nach Anspruch 5, wobei die Zusatzsignalisierungseinheit einen Farbstoff, ein Pigment, ein elektroaktives Molekül, einen fluoreszierenden Rest, aufregulierenden Phosphor oder einen mit Enzym verbundenen Signalisierungsrest, der Meerrettichperoxidase und alkalische Phosphatase beinhaltet, umfasst.

7. Verfahren nach Anspruch 5 oder 6, wobei die Signalisierungseinheit ein Metallocen, vorzugsweise Ferrocen oder ein Ferrocen-Derivat, umfasst.

8. Verfahren nach Anspruch 7, wobei die nicht-kolloidale Oberfläche ein magnetisches Kügelchen ist und das Bestimmen des Anheftens das magnetische Nahe-Heranziehen des Kügelchens mit einer Elektrode und das Bestimmen der Nähe des Metallocens zur Elektrode durch Aktivieren der Elektrode umfasst.

9. Verfahren nach einem der vorhergehenden Ansprüche, umfassend das Zulassen, dass sich mehrere Kolloidpartikel an die nicht-kolloidale Struktur anheften, und das Bestimmen des Anheftens der mehreren Partikel an die nicht-kolloidale Struktur, wobei die mehreren Kolloidpartikel vorzugsweise Zusatzsignalisierungseinheiten umfassen, wobei die Zusatzsignalisierungseinheiten vorzugsweise Farbstoffe, Pigmente oder elektroaktive Moleküle umfassen.

10. Verfahren nach einem der vorhergehenden Ansprüche, wobei der Stoff und der Bindungspartner biologisch aneinander binden.

11. Verfahren nach einem der vorhergehenden Ansprüche, wobei der Stoff und/oder der Bindungspartner ein Antikörper ist.

12. Verfahren nach einem der vorhergehenden Ansprüche, wobei der biologische oder chemische Stoff Folgendes ist:
a) ein Arzneimittelkandidat, und der Bindungspartner ist ein Wirkungsziel des Arzneimittelkandidaten,
b) eine Nukleinsäuresequenz,
c) ein Peptid oder
d) ein Protein.

13. Verfahren nach einem der vorhergehenden Ansprüche, umfassend das Bestimmen, vorzugsweise durch Sichtprüfung, der Immobilisierung des Partikels an der nicht-kolloidalen Struktur durch Bestimmen einer Änderung des Spektrums absorbierter oder durchgeleiteter elektromagnetischer Strahlung, die mit dem Partikel in Wechselwirkung steht.

14. Verfahren nach einem der vorhergehenden Ansprüche, umfassend das Zulassen der Fähigkeit des Kolloidpartikels, sich in Gegenwart eines Arzneimittelkandidaten zur Unterbrechung der Wechselwirkung zwischen dem Stoff und dem Bindungspartner an die nicht-kolloidale Struktur anzuheften.

15. Verfahren nach einem der vorhergehenden Ansprüche, wobei die nicht-kolloidale Struktur ein Kügelchen ist, ferner das Bereitstellen mehrerer Kügelchen, mehrerer biologischer oder chemischer Stoffe, die zur Verbindung mit den Kügelchen in der Lage sind, mehrerer Kolloidpartikel und mehrerer Bindungspartner der biologischen oder chemischen Stoffe, die zur Verbindung mit den Partikeln in der Lage sind, umfassend, wobei bei mindestens einigen der Stoffe und der Bindungspartner eine Fähigkeit zur Bindung aneinander vermutet wird, wobei das Verfahren das In-Kontakt-Bringen mindestens einiger der Kügelchen mit mindestens einigen der Partikeln und das Bestimmen der Immobilisierung der Partikeln an den Kügelchen umfasst.

16. Verfahren nach Anspruch 15, wobei die biologischen oder chemischen Stoffe Arzneimittelkandidaten sind und die Bindungspartner Wirkungsziele der Arzneimittelkandidaten sind, wobei das Verfahren Folgendes umfasst:
a) Bereitstellen mindestens eines ersten und eines zweiten Kügelchens an zwei getrennten Orten, die jeweils einen anderen immobilisierten Arzneimittelkandidaten tragen, In-Kontakt-Bringen der mehreren Kolloidpartikeln mit den mindestens zwei Kügelchen und Unterscheiden der Verbindung der Kolloidpartikeln mit dem ersten Kügelchen von der mit dem zweiten Kügelchen, wobei sich die mindestens zwei Kügelchen vorzugsweise getrennt voneinander in mindestens zwei verschiedenen Vertiefungen einer Mikrotiterplatte befinden, oder
b) Bereitstellen von mindestens zwei Kügelchen, die einen Arzneimittelkandidaten tragen, an zwei getrennten Orten, In-Kontakt-Bringen des ersten Kügelchens mit einer ersten Gruppe von Kolloidpartikeln, die ein erstes Wirkungsziel des Arzneimittelkandidaten tragen, und In-Kontakt-Bringen des zweiten Kügelchens mit einer zweiten Gruppe von Kolloidpartikeln, die ein zweites Wirkungsziel des Arzneimittelkandidaten tragen, und Unterscheiden der Verbindung der ersten Gruppe von Kolloidpartikeln mit dem ersten Kügelchen von der der zweiten Gruppe von Kolloidpartikeln mit dem zweiten Kügelchen.

17. Verfahren nach einem der vorhergehenden Ansprüche, umfassend:
Bereitstellen mindestens einer ersten und einer zweiten nicht-kolloidalen Struktur, umfassend Polymerkügelchen, und mindestens eines ersten und eines zweiten Stoffes, die mit dem ersten beziehungsweise dem zweiten Kügelchen verbunden sind,
Bereitstellen mehrerer Kolloidpartikel, die jeweils daran immobilisiert einen vermuteten Bindungspartner des ersten und/oder zweiten Stoffes tragen,
In-Kontakt-Bringen der Kügelchen mit den Partikeln und
Unterscheiden der Verbindung der Partikeln mit dem ersten Kügelchen von der mit dem zweiten Kügelchen,
wobei vorzugsweise bei dem ersten und dem zweiten Stoff, die mit dem ersten und dem zweiten Polymerkügelchen verbunden sind, eine biologische oder chemische Wechselwirkung mit dem Bindungspartner vermutet wird, und der Unterscheidungsschritt das Unterscheiden der Wechselwirkung zwischen dem ersten Stoff und dem Bindungspartner von der des zweiten Stoffes mit dem Bindungspartner umfasst.

18. Verfahren nach einem der vorhergehenden Ansprüche, umfassend:
Bereitstellen mehrerer nicht-kolloidaler Strukturen, Kügelchen umfassend, die jeweils daran immobilisiert den Stoff tragen,
Bereitstellen einer ersten Gruppe und einer zweiten Gruppe von Kolloidpartikeln, wobei die erste Gruppe jeweils daran immobilisiert einen ersten vermuteten Bindungspartner des Stoffes trägt und die zweite Gruppe jeweils daran immobilisiert einen zweiten vermuteten Bindungspartner des Stoffes trägt,
In-Kontakt-Bringen eines ersten Kügelchens mit der ersten Gruppe von Kolloidpartikeln und eines zweiten Kügelchens mit der zweiten Gruppe von Kolloidpartikeln und
Unterscheiden der Verbindung der ersten Gruppe von Kolloidpartikeln mit dem ersten Kügelchen von der der zweiten Gruppe von Kolloidpartikeln mit dem Kügelchen,
wobei vorzugsweise bei dem ersten und dem zweiten vermuteten Bindungspartner eine biologische oder chemische Wechselwirkung mit dem Stoff vermutet wird und der Unterscheidungsschritt das Unterscheiden der Wechselwirkung zwischen dem Stoff und dem ersten vermuteten Bindungspartner von der des Stoffes mit dem zweiten vermuteten Bindungspartner umfasst.

19. Verfahren nach einem der Ansprüche 1, 2 oder 5 bis 14, wobei die nicht-kolloidale Struktur eine Zelle ist.

20. Verfahren nach Anspruch 19, wobei der Stoff ein Rezeptor an einer Oberfläche der Zelle ist und der Bindungspartner ein Ligand für den Rezeptor ist.

21. Verfahren nach Anspruch 20, wobei der Ligand ein Peptid, Protein, Antikörper, Enzym oder eine chemische Spezies ist.

22. Verfahren nach einem der Ansprüche 19 bis 21, umfassend das Bestimmen der Immobilisierung des Kolloidpartikels an der Zelle:
a) durch Bestimmen einer Änderung des Spektrums absorbierter oder durchgeleiteter elektromagnetischer Strahlung, die mit dem Partikel in Wechselwirkung steht, vorzugsweise durch Sichtprüfung, oder
b) elektronische Voltammetrie, vorzugsweise über Wechselstrom.

23. Verfahren nach Anspruch 19, wobei die nicht-kolloidale Struktur eine Zelle ist, die an einer ihrer Oberflächen einen Rezeptor exponiert, ferner das Bereitstellen eines Liganden für den Rezeptor umfassend, der zur Verbindung mit dem Kolloidpartikel in der Lage ist, und In-Kontakt-Bringen des Liganden und des Kolloidpartikels mit der Zelle in Gegenwart eines Arzneimittelkandidaten zur Unterbrechung der Wechselwirkung zwischen dem Liganden und dem Rezeptor, und Bestimmen des Anheftens des Kolloidpartikels an die Zelle, wobei der Bestimmungsschritt vorzugsweise das Bestimmen der Hemmung des Anheftens des Kolloidpartikels an die Zelle beinhaltet, was die Wirksamkeit des Arzneimittelkandidaten bei der Unterbrechung der Wechselwirkung zwischen Rezeptor/Zielprotein anzeigt, vorzugsweise das Bereitstellen mindestens einer ersten und einer zweiten Zelle an getrennten Orten umfassend, das In-Kontakt-Bringen der ersten Zelle mit dem Liganden für einen Zellrezeptor der ersten Zelle und mit einem Kolloidpartikel, wobei der Ligand zur Verbindung mit dem Kolloidpartikel in der Lage ist, und mit einem ersten Arzneimittelkandidaten zur Unterbrechung der Wechselwirkung zwischen dem Liganden und dem Rezeptor, und Zugeben des Liganden und des Kolloids und eines zweiten Arzneimittelkandidaten zur Unterbrechung der Wechselwirkung zwischen dem Liganden und dem Rezeptor zu der zweiten Zelle und Unterscheiden der Verbindung des Kolloidpartikels mit der ersten Zelle von der mit der zweiten Zelle.

24. Verfahren nach Anspruch 19, wobei die nicht-kolloidale Struktur eine Zelle ist, die an einer ihrer Oberflächen einen Rezeptor exponiert, ferner Folgendes umfassend:
Bereitstellen von mindestens zwei der Zellen an getrennten Orten,
In-Kontakt-Bringen jeder der Zellen mit einem anderen Zielprotein für einen Zellrezeptor und einem Kolloidpartikel, der zur Verbindung mit dem jeweiligen Zielprotein in der Lage ist, und
Bestimmen des Anheftens der Kolloidpartikeln an die Zellen, was die Bindung der verschiedenen Zielproteine an die Zellrezeptoren anzeigt.

25. Verfahren nach einem der vorhergehenden Ansprüche, wobei ein Enzym mit der Fähigkeit zur Spaltung des Stoffes oder Bindungspartners anwesend ist.

26. Verfahren nach Anspruch 25, wobei ein Arzneimittelkandidat zur Dämpfung der Aktivität des Enzyms anwesend ist, wobei vorzugsweise der Bindungspartner ein Protein oder Peptid umfasst, das durch das Enzym abgespalten werden kann, wobei das Protein vorzugsweise zur Verbindung mit dem Kolloidpartikel und mit der nicht-kolloidalen Oberfläche in der Lage ist.

27. Verfahren nach Anspruch 1, wobei das Kolloidpartikel und die nicht-kolloidale Struktur mit einem Substrat für ein Enzym in Kontakt gebracht werden, das zur Verbindung mit der nicht-kolloidalen Struktur in der Lage ist, mit einer molekularen Spezies, die über Enzymaktivität mit dem Substrat verbunden werden kann und die zur Verbindung mit dem Kolloidpartikel in der Lage ist, und mit einem Enzym für das Substrat, vorzugsweise ferner das In-Kontakt-Bringen des Kolloidpartikels und der nicht-kolloidalen Struktur mit einem Arzneimittelkandidaten für die Dämpfung der Aktivität des Enzyms umfassend.

28. Verfahren nach Anspruch 27, wobei die nicht-kolloidale Struktur ein magnetisches Kügelchen ist und die Signalisierungseinheit ein Metallocen ist, ferner das magnetische Nahe-Heranziehen des Kügelchens an eine Elektrode und das bestimmen der Nähe des Metallocens zur Elektrode durch Aktivieren der Elektrode umfassend, wodurch die Wirksamkeit des Arzneimittelkandidaten bei der Dämpfung der Aktivität des Enzyms bestimmt wird.

29. Verfahren nach Anspruch 27, wobei die nicht-kolloidale Struktur eine Oberfläche einer Elektrode ist und das Kolloidpartikel eine immobilisierte elektroaktive Einheit trägt und wobei das Verfahren das Bestimmen der Nähe der elektroaktiven Einheit zur Elektrode durch Aktivieren der Elektrode umfasst, wodurch die Wirksamkeit des Arzneimittelkandidaten bei der Dämpfung der Aktivität des Enzyms bestimmt wird.

30. Zusammensetzung, umfassend:
einen biologischen oder chemischen Stoff, der mit einer nicht-kolloidalen Struktur verbunden ist, und
einen Bindungspartner des biologischen oder chemischen Stoffes, der über eine Verbindung aus metallbindender Markierung/Metall/Chelat nichtkovalent mit einem Kolloidpartikel verbunden ist,
wobei das Kolloidpartikel fähig ist, sich über spezifische Wechselwirkung zwischen dem biologischen oder chemischen Stoff und dem Bindungspartner an die nicht-kolloidale Struktur zu heften,
wobei das Kolloidpartikel mit einer selbstorganisierenden Monoschicht derivatisiert ist.

31. Zusammensetzung nach Anspruch 30, wobei an das Kolloidpartikel eine Signalisierungseinheit immobilisiert ist, wobei die Signalisierungseinheit vorzugsweise eine elektroaktive Einheit ist.

## Revendications

1. Procédé comprenant :
l'exposition d'un agent biologique ou chimique, qui est lié à une structure non colloïdale, à un partenaire de liaison de l'agent biologique ou chimique, qui est lié de façon non covalente à une particule colloïdale par une liaison marqueur capable de liaison avec les métaux/métal/chélate ;
le fait de laisser la particule colloïdale se fixer à la structure non colloïdale par interaction spécifique entre l'agent biologique ou chimique et le partenaire de liaison ; et
la détermination de la fixation de la particule colloïdale à la structure non colloïdale ;
dans lequel la particule colloïdale est modifiée avec une monocouche autoassemblée.

2. Procédé selon la revendication 1, dans lequel la particule colloïdale est une particule colloïdale d'or.

3. Procédé selon la revendication 1 ou 2, dans lequel la structure non colloïdale est une électrode ou une bille.

4. Procédé selon la revendication 3, dans lequel la structure non colloïdale est une bille, de préférence dans lequel la bille comprend un matériau polymère, de l'agarose, du TentaGel et/ou un matériau magnétique, de préférence dans lequel la bille est une bille de polystyrène.

5. Procédé selon l'une quelconque des revendications précédentes, dans lequel la particule colloïdale comprend une entité de signalisation auxiliaire.

6. Procédé selon la revendication 5, dans lequel l'entité de signalisation auxiliaire comprend un colorant, un pigment, une molécule électroactive, une fraction fluorescente, un luminophore de régulation positive ou une fraction de signalisation liée à une enzyme notamment la peroxydase du raifort et la phosphatase alcaline.

7. Procédé selon la revendication 5 ou 6, dans lequel l'entité de signalisation comprend un métallocène, de préférence du ferrocène ou un dérivé de ferrocène.

8. Procédé selon la revendication 7, dans lequel la surface non colloïdale est une bille magnétique et la détermination de la fixation comprend l'entraînement magnétique de la bille à proximité d'une électrode et la détermination de la proximité du métallocène et de l'électrode par activation de l'électrode.

9. Procédé selon l'une quelconque des revendications précédentes, comprenant le fait de laisser une pluralité de particules colloïdales se fixer à la structure non colloïdale et la détermination de la fixation de la pluralité de particules à la structure non colloïdale, de préférence dans lequel la pluralité de particules colloïdales comprend des entités de signalisation auxiliaires, de préférence dans lequel les entités de signalisation auxiliaires comprennent des colorants, des pigments ou des molécules électroactives.

10. Procédé selon l'une quelconque des revendications précédentes, dans le lequel l'agent et le partenaire de liaison se lient biologiquement l'un à l'autre.

11. Procédé selon l'une quelconque des revendications précédentes, dans lequel au moins l'un de l'agent et du partenaire de liaison est un anticorps.

12. Procédé selon l'une quelconque des revendications précédentes, dans lequel l'agent biologique ou chimique est :
(a) un candidat-médicament et le partenaire de liaison est une cible du candidat-médicament ;
(b) une séquence d'acide nucléique ;
(c) un peptide ; ou
(d) une protéine.

13. Procédé selon l'une quelconque des revendications précédentes, comprenant la détermination, de préférence par inspection visuelle, de l'immobilisation de la particule sur la structure non colloïdale par détermination d'un changement de spectre de rayonnement électromagnétique absorbé ou transmis interagissant avec la particule.

14. Procédé selon l'une quelconque des revendications précédentes, comprenant le fait de laisser à la particule colloïdale la possibilité de se fixer à la structure non colloïdale en présence d'un médicament candidat pour l'interruption de l'interaction entre l'agent et le partenaire de liaison.

15. Procédé selon l'une quelconque des revendications précédentes, dans lequel la structure non colloïdale est une bille, comprenant en outre l'utilisation d'une pluralité de billes, d'une pluralité d'agents biologiques ou chimiques adaptés à se lier aux billes, d'une pluralité de particules colloïdales et d'une pluralité de partenaires de liaison des agents biologiques ou chimiques adaptés à se lier aux particules, dans lequel au moins certains des agents et des partenaires de liaison sont supposés avoir l'aptitude à se lier les uns aux autres, le procédé comprenant l'exposition d'au moins certaines des billes à au moins certaines des particules et la détermination de l'immobilisation des particules sur les billes.

16. Procédé selon la revendication 15, dans lequel les agents biologiques ou chimiques sont des candidats-médicaments et les partenaires de liaison sont des cibles des candidats-médicaments, le procédé comprenant :
(a) l'utilisation d'au moins une première bille et une seconde bille en deux endroits séparés, portant chacune un candidat-médicament immobilisé différent, l'exposition de la pluralité de particules colloïdales aux au moins deux billes et la différenciation de la liaison des particules colloïdales à la première bille par rapport à la seconde bille, de préférence les au moins deux billes étant situées séparément dans au moins deux puits différents d'une plaque à plusieurs puits ; ou
(b) l'utilisation d'au moins deux billes portant un candidat-médicament dans deux endroits séparés, l'exposition de la première bille à un premier ensemble de particules colloïdales portant une première cible du candidat-médicament et l'exposition de la seconde bille à un second ensemble de particules colloïdales portant une seconde cible du candidat-médicament et la différenciation de la liaison du premier ensemble de particules colloïdales à la première bille par rapport au second ensemble de particules colloïdales à la seconde bille.

17. Procédé selon l'une quelconque des revendications précédentes, comprenant :
l'utilisation d'au moins une première structure non colloïdale et une seconde structure non colloïdale comprenant des billes polymères et au moins un premier agent et un second agent liés aux première et seconde billes, respectivement ;
l'utilisation d'une pluralité de particules colloïdales portant chacune immobilisé sur celles-ci un partenaire de liaison supposé des premier et/ou second agents ;
l'exposition des billes aux particules ; et
la différenciation de la liaison des particules à la première bille par rapport à la seconde bille ;
de préférence dans lequel les premier et second agents liés aux première et seconde billes polymères sont supposés avoir une interaction biologique ou chimique avec le partenaire de liaison et l'étape de différenciation comprend la différenciation de l'interaction entre le premier agent et le partenaire de liaison par rapport au second agent et le partenaire de liaison.

18. Procédé selon l'une quelconque des revendications précédentes, comprenant :
l'utilisation d'une pluralité de structures non colloïdales comprenant des billes portant chacune l'agent immobilisé sur celles-ci ;
l'utilisation d'un premier ensemble et d'un second ensemble de particules colloïdales, chacune du premier ensemble portant immobilisé sur celle-ci un premier partenaire de liaison supposé de l'agent et chacune du second ensemble portant immobilisé sur celle-ci un second partenaire de liaison supposé de l'agent ;
l'exposition d'une première bille au premier ensemble de particules colloïdales et d'une seconde bille au second ensemble de particules colloïdales ; et
la différenciation de la liaison du premier ensemble de particules colloïdales à la première bille par rapport au second ensemble de particules colloïdales à la bille ;
de préférence dans lequel les premier et second partenaires de liaison supposés sont supposés avoir une interaction biologique ou chimique avec l'agent et l'étape de différenciation comprend la différenciation de l'interaction entre l'agent et le premier partenaire de liaison supposé par rapport à l'agent et le second partenaire de liaison supposé.

19. Procédé selon l'une quelconque des revendications 1, 2 ou 5 à 14, dans lequel la structure non colloïdale est une cellule.

20. Procédé selon la revendication 19, dans lequel l'agent est un récepteur à une surface de la cellule et le partenaire de liaison est un ligand pour le récepteur.

21. Procédé selon la revendication 20, dans lequel le ligand est un peptide, une protéine, un anticorps, une enzyme ou une espèce chimique.

22. Procédé selon l'une quelconque des revendications 19 à 21, comprenant la détermination de l'immobilisation de la particule colloïdale à la cellule :
(a) par détermination d'un changement de spectre de rayonnement électromagnétique absorbé ou transmis interagissant avec la particule, de préférence par inspection visuelle ; ou
(b) électroniquement, de préférence par voltampérométrie en courant alternatif.

23. Procédé selon la revendication 19, dans lequel la structure non colloïdale est une cellule exposant un récepteur à une surface de celle-ci, comprenant en outre
l'utilisation d'un ligand pour le récepteur adapté à se lier à la particule colloïdale et l'exposition du ligand et de la particule colloïdale à la cellule en présence d'un médicament candidat pour la perturbation de l'interaction entre le ligand et le récepteur ; et
la détermination de la fixation de la particule colloïdale à la cellule,
de préférence, dans lequel l'étape de détermination comprend la détermination de l'inhibition de la fixation de la particule colloïdale à la cellule témoignant de l'efficacité du médicament candidat à perturber l'interaction récepteur/protéine cible,
de préférence, comprenant
l'utilisation d'au moins une première cellule et une seconde cellule dans des endroits séparés,
l'exposition de la première cellule au ligand pour un récepteur cellulaire de la première cellule et à une particule colloïdale, le ligand étant adapté à se lier à la particule colloïdale, et à un premier médicament candidat pour la perturbation de l'interaction entre le ligand et le récepteur et
l'ajout à la seconde cellule du ligand et du colloïde et d'un second médicament candidat pour la perturbation de l'interaction entre le ligand et le récepteur et
la différenciation de la liaison de la particule colloïdale à la première cellule par rapport à la seconde cellule.

24. Procédé selon la revendication 19, dans lequel la structure non colloïdale est une cellule exposant un récepteur à une surface de celle-ci, comprenant en outre :
l'utilisation d'au moins deux des cellules dans des endroits séparés ;
l'exposition de chacune des cellules à une protéine cible différente pour un récepteur cellulaire et à une particule colloïdale adaptée à se lier à la protéine cible respective ; et
la détermination de la fixation des particules colloïdales aux cellules témoignant de la liaison des différentes protéines cibles aux récepteurs cellulaires.

25. Procédé selon l'une quelconque des revendications précédentes, dans lequel une enzyme ayant l'aptitude à scinder l'agent ou le partenaire de liaison est présente.

26. Procédé selon la revendication 25, dans lequel un médicament candidat pour la modération de l'activité de l'enzyme est présent, de préférence, dans lequel le partenaire de liaison comprend une protéine ou un peptide qui peut être scindé par l'enzyme, de préférence, dans lequel la protéine est adaptée à se lier à la particule colloïdale et à la surface non colloïdale.

27. Procédé selon la revendication 1, dans lequel la particule colloïdale et la structure non colloïdale sont exposées à un substrat pour une enzyme adapté à se lier à la structure non colloïdale, à une espèce moléculaire pouvant se lier au substrat par une activité enzymatique adaptée à se lier à la particule colloïdale et à une enzyme pour le substrat, de préférence comprenant en outre l'exposition de la particule colloïdale et de la structure non colloïdale à un médicament candidat pour la modération de l'activité de l'enzyme.

28. Procédé selon la revendication 27, dans lequel la structure non colloïdale est une bille magnétique et l'entité de signalisation est un métallocène, comprenant en outre l'entraînement magnétique de la bille à proximité d'une électrode et la détermination de la proximité du métallocène et de l'électrode par activation de l'électrode ce qui détermine de cette manière l'efficacité du candidat-médicament à modérer l'activité de l'enzyme.

29. Procédé selon la revendication 27, dans lequel la structure non colloïdale est une surface d'une électrode et la particule colloïdale porte une entité électroactive immobilisée et le procédé comprend la détermination de la proximité de l'entité électroactive et de l'électrode par activation de l'électrode ce qui détermine de cette manière l'efficacité du candidat-médicament à modérer l'activité de l'enzyme.

30. Composition comprenant :
un agent biologique ou chimique, lié à une structure non colloïdale ; et
un partenaire de liaison de l'agent biologique ou chimique, lié de façon non covalente à une particule colloïdale par une liaison marqueur capable de liaison avec les métaux/métal/chélate ;
dans laquelle la particule colloïdale est capable de se fixer à la structure non colloïdale par interaction spécifique entre l'agent biologique ou chimique et le partenaire de liaison ;
dans laquelle la particule colloïdale est modifiée avec une monocouche autoassemblée.

31. Composition selon la revendication 30, dans laquelle une entité de signalisation est immobilisée sur la particule colloïdale, de préférence dans laquelle l'entité de signalisation est une entité électroactive.
